(19) Europäisches Patentamt
European Patent Office
Office européen des brevets

(11) Veröffentlichungsnummer: **0 139 917**
**A1**

(12) # EUROPÄISCHE PATENTANMELDUNG

(21) Anmeldenummer: 84109311.5

(22) Anmeldetag: 06.08.84

(51) Int. Cl.4: A 61 K 31/44
C 07 D 213/77

(30) Priorität: 09.08.83 CH 4333/83
09.09.83 CH 4931/83
07.03.84 CH 1121/84
09.03.84 CH 1169/84

(43) Veröffentlichungstag der Anmeldung:
08.05.85 Patentblatt 85/19

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Fuhrer, Walter, Dr.
Munzacherweg 11
CH-4402 Frenkendorf(CH)

(72) Erfinder: Hofbauer, Karl Günther, Prof. Dr.
Leimenstrasse 66
CH-4051 Basel(CH)

(72) Erfinder: Habicht, Ernst, Dr.
Bienenstrasse 13
CH-4104 Oberwil(CH)

(74) Vertreter: Zumstein, Fritz sen., Dr. et al,
Bräuhausstrasse 4
D-8000 München 2(DE)

(54) **Pharmazeutische Präparate, acylierte Hydrazinverbindungen, Verfahren zu deren Herstellung.**

(57) Die Erfindung betrifft pharmazeutische Präparate enthaltend acylierte Hydrazinverbindungen der Formel

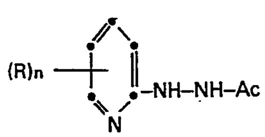

(I)

worin R einen gegebenenfalls substituierten aliphatischen oder aromatischen Kohlenwasserstoffrest und Ac einen Acylrest bedeutet, und n eine ganze Zahl von 1-4 darstellt, oder deren Salze, sowie neue Verbindungen dieser Art, oder deren Salze, Verfahren zur Herstellung solcher Verbindungen, pharmazeutische Präparate enthaltend solche neue Verbindungen und ihre Verwendung zur Herstellung von pharmazeutischen Präparaten.

Die pharmazeutischen Präparate als auch die neuen Verbindungen bewirken eine selektive renale Vasodilatation und sind dementsprechend zur Verwendung als renale Vasodilatatoren und Antihypertensiva geeignet.

EP 0 139 917 A1

BEZEICHNUNG GEÄNDERT
siehe Titelseite

4-14529/1-4/+

Acylierte Hydrazinverbindungen

Gegenstand der Erfindung sind pharmazeutische Präparate enthaltend acylierte Hydrazinverbindungen, ihre Verwendung als pharmakologisch aktive Präparate, neue Verbindungen dieser Art und Verfahren zu ihrer Herstellung, deren Verwendung zur Herstellung pharmazeutischer Präparate sowie als pharmakologisch aktive Verbindungen, und neue Ausgangsstoffe.

Die erfindungsgemässen pharmazeutischen Präparate enthalten Verbindungen der Formel

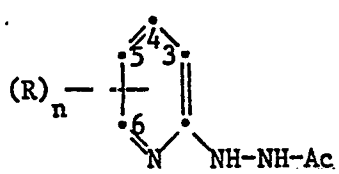

(I) ,

worin R einen gegebenenfalls substituierten aliphatischen oder aromatischen Kohlenwasserstoffrest und Ac einen Acylrest bedeutet, und n eine ganze Zahl von 1-4 darstellt, als Racematgemische, Racemate, optische Antipoden oder Salze solcher Verbindungen. Die Reste R können, sofern n einen Zahlenwert von 2-4 darstellt, gleich oder verschieden und in jeder der möglichen Stellungen am Pyridinring gebunden sein.

Die Erfindung betrifft ebenfalls die neuen Verbindungen der Formel I, worin R, n und Ac die unter der Formel I angegebenen Bedeutungen haben mit der Massgabe, dass R als in 3-Stellung gebundenes Niederalkyl 2-7 Kohlenstoffatome hat, und n den Wert 1 bedeutet, sofern Ac für die Acetylgruppe steht, und mit der weiteren Massgabe, dass R als in 3-, 4-, 5- oder 6-Stellung gebundenes Niederalkyl 2-7 Kohlenstoffatome hat und n den Wert 1 bedeutet, sofern Ac für Benzoyl steht, als Racematgemische, Racemate, optische Antipoden oder Salze solcher Verbindungen.

Ein gegebenenfalls substituierter aliphatischer Kohlenwasserstoffrest R ist z.B. gegebenenfalls substituiertes Niederalkyl, z.B. Niederalkyl, gegebenenfalls veräthertes oder verestertes Hydroxyniederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl, wie Aminoniederalkyl, N-Niederalkylaminoniederalkyl oder N,N-Diniederalkylaminoniederalkyl, Niederalkanoylaminoniederalkyl oder Niederalkoxycarbonylaminoniederalkyl, durch gegebenenfalls verestertes oder amidiertes Carboxy,wie Carboxy, Niederalkoxycarbonyl oder gegebenenfalls substituiertes Carbamoyl,wie Carbamoyl, N-Niederalkylcarbamoyl oder N,N-Diniederalkylcarbamyol oder auch durch eine Oxogruppe substituiertes Niederalkyl, wie Carboxyniederalkyl, Niederalkoxycarbonylniederalkyl, Carbamoylniederalkyl, N-Niederalkylcarbamoylniederalkyl oder N,N-Diniederalkylcarbamoylniederalkyl, oder auch Oxo-niederalkyl, ferner Cyanniederalkyl, gegebenenfalls substituiertes Arylniederalkyl, worin Aryl ein monocyclischer, aromatischer Kohlenwasserstoffrest, z.B. Phenyl, oder ein bicyclischer aromatischer Kohlenwasserstoffrest, wie 1- oder 2-Naphthyl oder teilweise gesättigtes Naphthyl, wie 1,2,3,4-Tetrahydro-5-naphthyl ist, worin Substituenten Niederalkyl, Hydroxy und/oder Halogen, gegebenenfalls veräthertes oder verestertes Hydroxyniederalkyl, z.B. wie angegeben, etwa Niederalkoxyniederalkyl oder Halogenniederalkyl, Niederalkoxy gegebenenfalls veräthertes oder verestertes Hydroxyniederalkoxy, wie Niederalkoxyniederalkoxy oder Halogenniederalkoxy, Niederalkenyloxy, Niederalkinyloxy, oder gegebenenfalls substituiertes, wie niederalkyliertes oder acyliertes Aminoniederalkyl, etwa wie oben

angegeben, z.B. Mono- oder Diniederalkylaminoniederalkyl oder Niederalkanoylaminoniederalkyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Amino, Niederalkanoylamino, Niederalkoxycarbonylamino, ferner Niederalkylamino oder Diniederalkylamino sein können, wobei solche Substituenten gleich oder verschieden und einfach oder mehrfach, insbesondere 1-3 fach vorhanden sein können.

Ein gegebenenfalls substituierter aromatischer Kohlenwasserstoffrest R ist ein mono- oder bicyclischer, carbocyclischer oder heterocyclischer aromatischer Kohlenwasserstoffrest, z.B. Phenyl, 1- oder 2-Naphthyl oder teilweise gesättigtes Naphthyl, wie 1,2,3,4-Tetrahydro-5-naphthyl. Als mono- oder bicyclisches Heteroaryl stellt R einen über ein Ringkohlenstoffatom mit dem Pyridylrest verbundenen, ein Sauerstoff-, Schwefel oder Stickstoffatom und gegebenenfalls 1-3, zusätzliche Stickstoffatome als Ringglieder enthaltenden Rest, vorzugsweise mit 5 oder 6 Ringgliedern dar, der mindestens partiell hydriert und in diesem Fall durch Oxo substituiert sein kann, wobei in einem bicyclischen heterocyclischen Rest der zweite Ring ein ankondensierter Benzoring sein kann, und bedeutet insbesondere Pyridyl, z.B. 2-, 3- oder 4-Pyridyl, oder Dihydro-oxo-pyridinyl z.B. 1,6-Dihydro-6-oxo-2-pyridinyl, Pyridazinyl, z.B. 3-Pyridazinyl, Pyrazinyl, z.B. 2-Pyrazinyl, Pyrimidinyl, z.B. 2-, 4- oder 5-Pyrimidinyl, oder Dihydro-oxo-pyrimidinyl z.B. 3,4-Dihydro-4-oxo-2-pyrimidinyl, Furyl z.B. 2- oder 3-Furyl, Pyrryl z.B. 2- oder 3-Pyrryl, Thienyl z.B. 2- oder 3-Thienyl, Oxazolyl z.B. 2- oder 4-Oxazolyl, Thiazolyl, z.B. 2-, 4- oder 5-Thiazolyl, Thiadiazolyl z.B. 1,2,4-Thiadiazol-3- oder -5-yl oder 1,2,5-Thiadiazol-3-yl, Imidazolyl z.B. Imidazol-2- oder -4-yl, Pyrazolyl z.B. 3- oder 4-Pyrazolyl, Triazolyl z.B. 1,2,3-Triazol-4-yl oder 1,2,4-Triazol-3-yl, Tetrazolyl wie Tetrazol-5-yl, ferner

Indolyl z.B. Indol-4-yl, Chinolinyl, z.B. 2-Chinolinyl, Isochinolinyl, z.B. 1-Isochinolinyl, Benzimidazolyl, z.B. 2-Benzimidazolyl, Benzofuranyl, z.B. 4- oder 5-Benzofuranyl, Benzothienyl z.B. 4- oder 5-Benzothienyl, oder Naphthyridinyl z.B. 1,8-Naphthyridin-2-yl.

Substitutenten eines mono- oder bicyclischen, carbocyclischen oder heterocyclischen Arylrestes R, wobei im letzterem Falle in erster Linie ein Ringkohlenstoffatom, aber auch ein sekundäres Ringstickstoffatom substituiert sein kann, und solche Substituenten ein- oder mehrfach, vorzugsweise höchstens vierfach vorhanden sein können, sind z.B. gegebenenfalls substituiertes Niederalkyl, z.B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie acyliertes Aminoniederalkyl, wie Niederalkanoylaminoniederalkyl oder Niederalkoxycarbonylaminoniederalkyl, oder Niederalkenyl oder Niederalkinyl, gegebenenfalls veräthertes oder verestertes Hydroxy oder Mercapto, wie Hydroxy, Niederalkoxy, Phenylniederalkoxy, Halogen, Mercapto, Niederalkylthio, durch gegebenenfalls veräthertes oder verestertes Hydroxy oder Mercapto oder durch Acyl substituiertes Niederalkoxy, z.B. Niederalkoxy, Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkinyloxy, Acyl, z.B. Niederalkanoyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Niederalkanoylamino, Niederalkoxycarbonylamino, Niederalkylsulfonyl, Sulfamoyl, gegebenenfalls substituiertes Ureido, ferner Amino, N-Niederalkylamino oder N,N-Diniederalkylamino.

Ein Acylrest Ac entspricht z.B. der Formel $-C(=O)-R_1$, worin $R_1$ einen gegebenenfalls substituierten aliphatischen, cycloaliphatischen, aromatischen oder araliphatischen Kohlenwasserstoffrest bedeutet, wie gegebenenfalls substituiertes Niederalkyl, Niederalkenyl oder Niederalkinyl, gegebenenfalls substituiertes Phenylniederalkyl, Phenylniederalkenyl oder Phenylniederalkinyl, gegebenenfalls substituiertes mono- oder polycyclisches Cycloalkyl, oder als aromatischer Rest für einen gegebenenfalls substituierten mono- oder bicyclischen, carbocyclischen oder heterocyclischen Arylrest, z.B. wie oben für die Gruppe R definiert, und dementsprechend beispielsweise für Phenyl oder Pyridyl steht.

Substituenten von Niederalkyl, Niederalkenyl oder Niederalkinyl sowie Phenylniederalkyl, Phenylniederalkenyl oder Phenylniederalkinyl sowie mono- oder polycyclisches Cycloalkyl, etwa Cycloalkyl, sind z.B. gegebenenfalls veräthertes oder verestertes Hydroxy, wie Hydroxy, Niederalkoxy und/oder Halogen, gegebenenfalls substituiertes wie niederalkyliertes oder acyliertes Amino, wie Amino, N-Niederalkylamino oder N,N-Diniederalkylamino, Niederalkanoylamino und/oder gegebenenfalls funktionell abgewandeltes Carboxy, wie Carboxy, verestertes Carboxy, z.B. Niederalkoxycarbonyl, gegebenenfalls substituiertes Carbamoyl, Cyan oder Nitro, wobei substituierte Reste einen oder mehrere, insbesondere bis zu drei Substituenten, die gleich oder verschieden sein können in irgendwelchen der zur Substitution geeigneten Stellungen aufweisen können.

Substituenten eines mono- oder bicyclischen, carbocyclischen oder heterocyclischen Arylrestes entsprechen den für die Gruppe R angegebenen Substituenten, beispielsweise gegebenenfalls substituierte aliphatische Kohlenwasserstoffreste, wie gegebenenfalls substituiertes Niederalkyl.

Ein Acylrest Ac ist ferner der Rest eines Monoesters oder Monoamids der Kohlensäure, wie unsubstituiertes oder substituiertes, z.B. freies oder veräthertes Hydroxy, wie Niederalkoxy, oder unsubstituiertes oder substituiertes Amino, wie Amino, Niederalkylamino, und in erster Line disubstituiertes Amino, wie Diniederalkylamino, N-Niederalkyl-N-phenylniederalkyl-amino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder Halogen substituiertes Phenyl enthaltendes Niederalkoxycarbonyl, ferner N-unsubstituiertes oder N-mono- oder N,N-disubstituiertes Carbamoyl, wie N-Niederalkylcarbamoyl, N,N-Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylen-carbamoyl.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Niederalkyl ist z.B. Methyl, Aethyl, n-Propyl, Isopropyl, n-Butyl, Isobutyl oder tert.-Butyl; dementsprechend ist Niederalkyl mit 2-7 Kohlenstoffatomen z.B. Aethyl, n-Propyl, oder n-Butyl; substituiertes Niederalkyl ist insbesondere entsprechenes Methyl oder 1- oder 2-Aethyl.

Niederalkenyl ist z.B. Vinyl, Allyl, 2- oder 3-Methallyl oder 3,3-Dimethylallyl.

Niederalkinyl ist insbesondere Propargyl.

Niederalkoxy ist z.B. Methoxy, Aethoxy, n-Propyloxy, Isopropyloxy, n-Butyloxy oder Isobutyloxy, während Phenylniederalkoxy z.B. Benzyloxy oder 1- oder 2-Phenyläthoxy, Niederalkenyloxy z.B. Allyloxy, 2- oder 3-Methallyloxy oder 3,3-Dimethylallyloxy und Niederalkinyloxy insbesondere Propargyloxy darstellt.

Niederalkylthio ist z.B. Methylthio, Aethylthio, n-Propylthio oder Isopropylthio.

Niederalkylsulfonyl ist z.B. Methylsulfonyl oder Aethylsulfonyl.

Halogen ist vorzugsweise Halogen mit Atomnummer bis zu 35, d.h. Fluor, Chlor oder Brom.

Niederalkanoyl ist z.B. Acetyl, Propionyl oder Butyryl.

Gegebenenfalls substituiertes Niederalkoxycarbonyl ist z.B. durch gegebenenfalls über ein Sauerstoffatom gebundenes Acyl, z.B. Niederalkanoyl wie Acetyl oder Pivaloyl, Niederalkoxycarbonyl, wie Methoxy- oder Aethoxycarbonyl, oder Aroyl, wie Benzol, substituiertes Niederalkoxycarbonyl, und ist z.B. Methoxycarbonyl, Aethoxycarbonyl, 1- oder 2-(Acetyloxy)-äthoxycarbonyl, Pivaloyloxymethoxycarbonyl, 1- oder 2-(Methoxycarbonyloxy)-äthoxycarbonyl oder 1- oder 2-(Benzoyloxy)-äthoxycarbonyl.

Gegebenenfalls substituiertes Carbamoyl ist z.B. Carbamoyl, oder N-Niederalkyl- oder N,N-Diniederalkylcarbamoyl, wie N-Methylcarbamoyl, N,N-Dimethylcarbamoyl, N-Aethylcarbamoyl oder N,N-Diäthylcarbamoyl.

Aminoniederalkyl ist z.B. Aminomethyl oder 2-Aminoäthyl.

Oxy-niederalkyl ist z.B. 2-Oxo-n-butyl.

Niederalkanoylamino ist z.B. Acetylamino oder Propionylamino.

Niederalkoxycarbonylamino ist z.B. Methoxycarbonylamino oder Aethoxycarbonylamino.

Gegebenenfalls substituiertes Ureido ist z.B. Ureido oder 3-Niederalkyl- oder 3-Cycloalkyl-ureido, worin Cycloalkyl z.B. 5-7 Ringglieder enthält, z.B. 3-Methylureido, 3-Aethylureido oder 3-Cyclohexylureido.

N-Niederalkylamino und N,N-Diniederalkylamino sind z.B. Methylamino, Aethylamino, Dimethylamino oder Diäthylamino.

Hydroxyniederalkyl ist vorzugsweise Hydroxymethyl oder 1- und in erster Linie 2-Hydroxyäthyl.

Niederalkoxyniederalkyl ist vorzugsweise Niederalkoxymethyl oder 1- und in erster Linie 2-Niederalkoxyäthyl, z.B. Methoxymethyl, Aethoxymethyl, 2-Methoxy-äthyl oder 2-Aethoxy-äthyl.

Halogenniederalkyl ist vorzugsweise Halogenmethyl, z.B. Trifluormethyl.

Hydroxyniederalkoxy ist z.B. Hydroxymethoxy oder 2-Hydroxyäthoxy.

Niederalkoxyniederalkoxy ist z.B. 2-Methoxyäthoxy oder 2-Aethoxyäthoxy.

Niederalkanoylaminoniederalkyl ist insbesondere Niederalkanoylaminomethyl oder 1- und in erster Linie 2-Niederalkanoylamino-äthyl, z.B. Acetylaminomethyl, 2-Acetylamino-äthyl oder 2-Propionylamino-äthyl.

Niederalkoxycarbonylaminoniederalkyl ist insbesondere Niederalkoxycarbonylaminomethyl, oder 1- und in erster Linie 2-Niederalkoxycarbonylamino-äthyl, z.B. Methoxycarbonylaminomethyl, 2-Methoxycarbonylamino-äthyl oder 2-Aethoxycarbonylamino-äthyl.

Carboxyniederalkyl ist z.B. 2-Carboxyäthyl.

Niederalkoxycarbonylniederalkyl ist z.B. Methoxycarbonylmethyl oder 2-Aethoxycarbonyläthyl.

Cyanniederalkyl ist z.B. Cyanmethyl.

Niederalkoxyniederalkoxy ist u.a. Niederalkoxymethoxy oder 1- und insbesondere 2-Niederalkoxy-äthoxy, z.B. Methoxymethoxy, 2-Methoxy-äthoxy oder 2-Aethoxy-äthoxy.

Niederalkylthioniederalkoxy ist insbesondere Niederalkylthiomethoxy oder 1- und in erster Linie 2-Niederalkylthioäthoxy, z.B. 2-Methylthio-äthoxy oder 2-Aethylthio-äthoxy.

Halogenniederalkoxy ist insbesondere 2-Halogenäthoxy, z.B. 2-Chloräthoxy.

Niederalkanoylniederalkoxy ist z.B. Niederalkanoylmethoxy oder 1- oder 2-Niederalkanoyläthoxy, z.B. Acetylmethoxy.
Phenylniederalkyl ist z.B. Benzyl oder 2-Phenyläthyl.

N-Niederalkyl-N-phenylniederalkyl-amino ist z.B. N-Methyl-N-benzylamino oder N-methyl-N-(2-phenyläthyl)-amino.

Phenylniederalkenyl ist z.B. Phenylvinyl oder 1-Phenyl-2-propenyl.
Phenylniederalkinyl ist z.B. 1-Phenyl-2-propinyl.

Niederalkylenamino enthält vorzugsweise 4 bis 6 Ringkohlenstoffatome und ist z.B. Pyrrolidino oder Piperidino, während durch Sauerstoff unterbrochenes Niederalkylenamino z.B. Morpholino, wie 4-Morpholino, durch Schwefel unterbrochenes Niederalkylenamino z.B. Thiomorpholino, wie 4-Thiomorpholino, und durch gegebenenfalls, z.B. durch Niederalkyl, substituierten Stickstoff unterbrochenes Niederalkylenamino z.B. Piperazino oder 4-Methylpiperazino darstellen kann.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholino-carbonyl, 1-Piperazinocarbonyl oder 4-Methyl-1-piperazino-carbonyl bedeutet.

Monocyclisches Cycloalkyl einschliesslich polycyclisches Cycloalkyl enthält vorzugsweise 3-10 Kohlenstoffatome und ist z.B. Cyclopropyl, Cyclopentyl oder Cyclohexyl, während polycyclisches Cycloalkyl z.B. Bicyclo[2,2,1]heptyl (Norbornyl), Bicyclo[2,2,2]octyl oder Adamantyl, wie 1-Adamantyl ist.

Die neuen Verbindungen können in Form ihrer Salze, wie ihrer Säureadditionssalze und in erster Linie ihrer pharmazeutisch verwendbaren nicht-toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäure, oder Phosphorsäure, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-,

Aethansulfon-, 2-Hydroxyäthansulfon-, Aethylensulfon-, Toluolsulfon-, Naphthalinsulfon- oder Sulfanilsäure, oder mit anderen sauren organischen Stoffen, wie Ascorbinsäure. Salze sind ferner solche, die mit Basen, insbesondere pharmazeutisch verwendbaren, nicht-toxischen Basen und saure Gruppen, z.B. Carboxygruppen enthaltenden Verbindungen der Formel I gebildet sind. Geeignete Basen sind z.B. solche anorganischer Natur, wie die Carbonate oder Hydroxide von Alkalimetall- oder Erdalkalimetallen, z.B. Carbonate oder Hydroxide des Natriums, Kaliums, Magnesiums oder Calciums, ferner Ammoniak, oder organische Basen, z.B. geeignete Stickstoffbasen, etwa geeignete Amine, wie gegebenenfalls substituierte, z.B. durch Hydroxy substituierte primäre, sekundäre oder tertiäre, aliphatische oder cyclische Amine, wie z.B. Triäthylamin, N-Aethylpiperidin, Di- oder Triäthanolamin, Pyrrolidin, N-(2-Hydroxyäthyl)-pyrrolidin, Piperazin, N-(2-Hydroxyäthyl)-piperazin, ferner N-Methylglucosamin.

Die erfindungsgemässen pharmazeutischen Präparate, enthaltend acylierte Hydrazinverbindungen der Formel I als auch die neuen Verbindungen der Formel I besitzen wertvolle pharmakologische Eigenschaften. Insbesondere wirken sie in spezifischer Weise auf die Nierendurchblutung und die Natriumausscheidung. So bewirken sie eine selektive renale Vasodilatation, wie durch hämodynamische Messungen an Ratten gezeigt werden kann. So kann an narkotisierten Ratten, deren Blutdruck und Nierendurchblutung kontinuierlich gemessen werden (elektromagnetisches Flowmeter), nach i.v. Verabreichung von etwa 3 mg/kg bis etwa 15 mg/kg solcher Substanzen, insbesondere solcher Verbindungen der Formel I, worin Ac den $\gamma$-Glutamylrest bedeutet, eine renale Vasodilatation ohne Blutdrucksenkung beobachtet werden, während nach Applikation sehr viel höherer Dosen, etwa von 70 mg/kg i.v. eine Blutdrucksenkung von etwa -20 mm/Hg auftritt. An Ratten, bei denen

gleichzeitig das Herzminutenvolumen (Thermodilution), die Durchblutung der Niere, des Mesenterialgebiets und der Hinterextremität (elektromagnetisches Flowmeter) gemessen werden, führt die i.v.-Verabreichung einer Dosis von 4 mg/kg solcher Verbindungen zu einer etwa 25%-igen Verminderung des renalen Gefässwiderstands, während des mesenteriale, femorale und der totale periphere Gefässwiderstand unverändert bleiben. In ähnlichen Versuchen an wachen, spontan hypertensiven Ratten mit chronisch implantierten Doppler-Ultraschall-Flussmessköpfen kann ebenfalls gezeigt werden, dass solche Verbindungen in einem Dosenbereich von etwa 3 mg/kg bis etwa 10 mg/kg i.v. den renalen Gefässwiderstand senken, während der Gefässwiderstand im Mesenterialgebiet und in der Hinterextremität nicht beeinflusst wird. Erst deutlich höhere Dosen, (etwa 30 mg/kg i.v.) führen zu einem Blutdruckabfall und einer Vasodilatation in allen drei untersuchten Gefässgebieten. Erst höhere Dosen bewirken nach i.v.-Applikation von etwa 30 mg/kg oder p.o.-Verabreichung von 60 mg/kg an wachen Ratten einen während mindestens 4 Stunden anhaltenden Blutdruckabfall von etwa -25 mg/Hg. Nach i.v.-Verabreichung von Dosen etwa im Bereich von 15 mg/kg an Ratten führen diese Verbindungen zu einer während 3 Stunden anhaltenden Zunahme der Natriumausscheidung. In Versuchen an narkotisierten Ratten kann gezeigt werden, dass insbesondere solche Verbindungen der Formel I, worin Ac den γ-Glutamylrest bedeutet, in einer Dosis von 10 mg/kg i.v. zu einer Erhöhung der glomerulären Filtrationsrate (gemessen als Inulin-Clearance) um etwa 50 % führt. Daraus lässt sich ableiten, dass solche Verbindungen eine vorwiegend präglomeruläre Vasodilation bewirken. Die chronische perorale Gabe solcher Verbindungen in einer Dosis von 10 mg/kg 2-mal täglich während 3 Wochen führt an Ratten mit spontaner Hypertonie zu einer anhaltenden Blutdrucksenkung um etwa -20 bis -25 mm Hg, wobei die Herzfrequenz unbeeinflusst bleibt. Aehnliche Wirkungen können unter Behand-

lung mit 10 mg/kg i.v. pro Tag, verabreicht über osmotische Minipumpen während einer Woche, beobachtet werden. Verbindungen der Formel I, worin Ac den γ-L-Glutamyl-Rest bedeutet, werden in der Niere durch die γ-Glutamyl-trans-Peptidase gespalten. Darüberhinaus lassen sich ausgeprägte blutdrucksenkende Wirksamkeiten, insbesondere an solchen Verbindungen der Formel I feststellen, worin Ac den Rest eines Monoesters der Kohlensäure bedeutet. Insbesondere Verbindungen dieser Art bewirken an renal hypertensiven männlichen Ratten (Goldblatt-Methode) eine ausgeprägte Blutdrucksenkung, die auf indirektem Wege an nicht-anaesthesierten Ratten nach der Methode von Gerold et.al. (Helv. Physiol. Acta 24, 58, (1966) ermittelt wird. So kann nach täglicher peroraler Verabreichung solcher Verbindungen während 4 Tagen, wobei das Ausmass der Blutdrucksenkung am 4. Tag, 2 Stunden nach Verabreichung der letzten Dosis ermittelt wird, bei einer Tagesdosis von 3 mg/kg eine Blutdrucksenkung von -27 mm Hg, bei einer Tagesdosis von 10 mg/kg eine Blutdrucksenkung von -81 mm Hg und bei einer Tagesdosis von 100 mg/kg eine Blutdrucksenkung von -108 mm Hg festgestellt werden.

Aufgrund dieser Eigenschaften erscheinen die pharmazeutischen Präparate enthaltend Verbindungen der Formel I oder die neuen Verbindungen der Formel I zur Verwendung als renale Vasodilatatoren und als Antihypertensiva geeignet.

Verbindungen der Formel I, worin Ac den Pyridylcarbonylrest bedeutet, entfalten ausserdem antileukämische Wirksamkeit, wie beispielsweise an Mäusen mit durch i.p. Transplantation verursachter Leukämie P 388 gezeigt werden kann, indem nach während 5 aufeinander folgenden Tagen jeweils 1 mal täglich erfolgter i.p. Verabreichung von 100 mg/kg solcher Verbindungen der Quotient T/C 129 %, bzw. nach während 5 aufeinander folgenden

Tagen jeweils 1 mal täglich erfolgter i.p. Verabreichung von 50 mg/kg solcher Verbindungen der Quotient T/C 125 % beträgt. An der durch i.p. Transplantation an Mäusen verursachten Leukämie L 1210 kann nach während 9 aufeinander folgenden Tagen jeweils 1 mal täglich erfolgter i.p. Verabreichung von 100 mg/kg solcher Verbindungen der Wert des Quotienten T/C mit 151 %, bzw. nach auf die gleiche Weise und Dauer erfolgter i.p. Gabe von 60 mg/kg solcher Verbindungen mit 123 % festgestellt werden. (Diese Befunde wurden in Testmodellen als Developmental Therapeutics Program, Division of Cancer Treatment, National Cancer Institute, Bethesda, Maryland (USA) im Auftrag der Anmelderin erhoben). Aufgrund dieser Eigenschaften erscheinen Verbindungen der Formel I, worin Ac den Pyridylcarbonylrest bedeutet, zur Behandlung der Leukämie geeignet.

Die neuen Verbindungen der Formel I können auch als wertvolle Zwischenprodukte für die Herstellung anderer wertvoller, insbesondere pharmazeutisch wirksamer Verbindungen verwendet werden.

Die Erfindung betrifft insbesondere pharmazeutische Präparate enthaltend Verbindungen der Formel I, worin n eine ganze Zahl von 1-4 darstellt und R gegebenenfalls substituiertes Niederalkyl, Niederalkenyl oder Niederalkinyl, oder einen mono- oder bicyclischen, carbocyclischen oder heterocyclischen aromatischen Kohlenwasserstoffrest bedeutet, Ac einen Rest der Formel $-C(=O)-R_1$ darstellt, worin $R_1$ einen gegebenenfalls substituierten aliphatischen, cycloaliphatischen, aromatischen oder araliphatischen Kohlenwasserstoffrest, oder Ac den Rest eines Monoesters oder Monoamids der Kohlensäure bedeutet,

als Racematgemische, Racemate, optische Antipoden oder deren Salze.

Die Erfindung betrifft insbesondere pharmazeutische Präparate enthaltend Verbindungen der Formel I, worin n eine ganze Zahl von 1-4 darstellt und R Niederalkyl, gegebenenfalls veräthertes oder verestertes Hydroxyniederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl, wie Aminoniederalkyl, N-Niederalkylaminoniederalkyl oder N,N-Diniederalkylaminoniederalkyl, Niederalkanoylaminoniederalkyl oder Niederalkoxycarbonylaminoniederalkyl, durch gegebenenfalls verestertes oder amidiertes Carboxy, wie Carboxy, Aethoxycarbonyl oder gegebenenfalls substituiertes Carbamoyl, wie Carbamoyl, substituiertes Niederalkyl, wie Carboxyniederalkyl, Niederalkoxycarbonylniederalkyl, Carbamoylniederalkyl, Oxo-niederalkyl, ferner Cyanniederalkyl, gegebenenfalls substituiertes Arylniederalkyl, worin Aryl ein monocyclischer, aromatischer Kohlenwasserstoffrest, z.B. Phenyl, oder ein bicyclischer aromatischer Kohlenwasserstoffrest, wie 1- oder 2-Naphthyl, oder teilweise gesättigtes Naphthyl, wie 1,2,3,4-Tetrahydro-5-naphthyl ist, wobei Substituenten Niederalkyl, Hydroxy und/oder Halogen, Hydroxyniederalkyl, Niederalkoxyniederalkyl, Halogenniederalkyl, Niederalkoxy, Niederalkenyloxy, oder gegebenenfalls niederalkyliertes oder acyliertes Aminoniederalkyl, z.B. Mono- oder Diniederalkylaminoniederalkyl oder Niederalkanoylaminoniederalkyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls acyliertes Amino, z.B. Amino, Niederalkylamino, Niederalkoxycarbonylamino, Niederalkylamino oder Diniederalkylamino sein und solche Substituenten gleich oder verschieden und 1-3-fach vorhanden sein können, oder R und das in der Gruppe Ac enthaltene $R_1$ jeweils für Aryl, wie Phenyl, 1- oder 2-Naphthyl oder teilweise gesättigtes Naphthyl, wie 1,2,3,4-Tetrahydro-5-naphthyl oder einen über ein Ringkohlenstoffatom mit dem Pyridylrest verbundenen, ein Sauerstoff-, Schwefel- oder Stickstoffatom und gegebenenfalls 1-3 zusätzliche Stickstoffatome als Ringglieder enthaltenden Rest, vorzugsweise mit 5 oder 6 Ringgliedern steht, der mindestens partiell hydriert und in diesem Fall durch Oxo substituiert sein kann, wobei in einem bicyclischen heterocyclischen Rest der zweite Ring ein ankondensierter Benzoring sein kann, und insbesondere Pyridyl, z.B. 2-, 3- oder 4-Pyridyl, oder Dihydro-oxo-pyridyl z.B. 1,6-Dihydro-6-oxo-2-pyridinyl, Pyridazinyl, z.B. 3-Pyridazinyl, Pyrazinyl, z.B. 2-Pyrazinyl, Pyrimidinyl, z.B. 2-, 4- oder 5-Pyrimidinyl, oder Dihydro-oxo-pyrimidinyl z.B. 3,4-Dihydro-4-oxo-2-pyrimidinyl, Furyl z.B. 2- oder 3-Furyl, Pyrryl z.B. 2- oder 3-Pyrryl, Thienyl z.B. 2- oder 3-Thienyl, Oxazolyl z.B. 2- oder 4-Oxazolyl, Thiazolyl, z.B. 2-, 4- oder 5-Thiazolyl, Thiadiazolyl z.B. 1,2,4-Thiadiazol-3- oder -5-yl oder 1,2,5-Thiadiazol-3-yl, Imidazolyl z.B. Imidazol-2- oder -4-yl, Pyrazolyl z.B. 3- oder 4-Pyrazolyl, Triazolyl, z.B. 1,2,3-Triazol-4-yl oder 1,2,4-Triazol-3-yl, Tetrazolyl wie Tetrazol-5-yl, ferner Indolyl z.B. Indol-4-yl, Chinolinyl, z.B. 2-Chinolinyl, Isochinolinyl, z.B. 1-Isochinolinyl, Benzimidazolyl, z.B. 2-Benzimidazolyl, Benzofuranyl, z.B. 4- oder 5-Benzofuranyl, Benzothienyl, z.B. 4- oder 5-Benzothienyl, oder Naphthyridinyl z.B. 1,8-Naphthyridin-2-yl bedeutet, wobei solche Arylreste in erster Linie an einem Ringkohlenstoffatom, aber auch einem sekundären Ringstickstoffatom stehen und ein- oder mehrfach, vorzugsweise höchstens vierfach, durch gegebenenfalls substituiertes Niederalkyl, z.B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl wie Aminoniederalkyl, N-Niederalkylaminoniederalkyl, N,N-Diniederalkylaminoniederalkyl, Niederalkanoylaminoniederalkyl oder Niederalkoxycarbonylaminoniederalkyl,

oder Niederalkenyl oder Niederalkinyl, gegebenenfalls veräthertes oder verestertes Hydroxy oder Mercapto, wie Hydroxy, Niederalkoxy, Phenylniederalkoxy, Halogen, Mercapto, Niederalkylthio, Phenylniederalkylthio, durch gegebenenfalls veräthertes oder verestertes Hydroxy oder Mercapto,oder durch Acyl substituiertes Niederalkoxy, z.B. Niederalkoxy, Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkinyloxy, Niederalkylthio, Acyl, z.B. Niederalkanoyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Niederalkanoylamino, Niederalkoxycarbonylamino, Niederalkylsulfonyl, Sulfamoyl, gegebenenfalls substituiertes Ureido, ferner Amino, N-Niederalkylamino oder N,N-Diniederalkylamino, substituiert sein können, das in der Gruppe Ac enthaltene $R_1$ ausserdem gegebenenfalls durch gegebenenfalls veräthertes oder verestertes Hydroxy, wie Hydroxy, Niederalkoxy und/oder Halogen, gegebenenfalls substituiertes wie niederalkyliertes oder acyliertes Amino, wie Amino, N-Niederalkylamino oder N,N-Diniederalkylamino, Niederalkanoylamino und/oder gegebenenfalls funktionell abgewandeltes Carboxy, wie Carboxy, verestertes Carboxy, z.B. gegebenenfalls substituiertes Niederalkoxycarbonyl, gegebenenfalls substituiertes Carbamoyl, oder Cyan, wobei substituierte Reste einen oder mehrere Substituenten die gleich oder verschieden und 1-3-fach vorhanden sein können, in irgendwelchen der zur Substitution geeigneten Stellungen aufweisen können, substituiertes Niederalkyl, Niederalkenyl, Niederalkinyl, Phenylniederalkyl, Phenylniederalkenyl, Phenylniederalkinyl, mono- oder polycyclisches Cycloalkyl, etwa Cycloalkyl, bedeutet, oder Ac ausserdem unsubstituiertes oder substituiertes, z.B. freies oder veräthertes Hydroxy, wie Niederalkoxy, oder unsubstituiertes oder substituiertes Amino, wie Amino, Niederalkylamino, und in erster Linie disubstituiertes Amino, wie Diniederalkylamino, N-Niederalkyl-N-phenylniederalkylamino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder Halogen substituiertes Phenyl enthaltendes Niederalkoxycarbonyl, ferner N-unsubstituiertes oder N-mono- oder N,N-disubstituiertes Carbamoyl, wie Niederalkylcarbamoyl, Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylencarbamoyl darstellt, als Racematgemische, Racemate, optische Antipoden oder deren Salze.

Die Erfindung betrifft insbesondere pharmazeutische Präparate enthaltend Verbindungen der Formel I, worin n für 1 oder 2 steht und R Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. Acetylaminomethyl bedeutet, und $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. 2-Acetylaminoäthyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Niederalkanoylamino, z.B. Acetylamino, oder Sulfamoyl substituiertes Phenyl oder Pyridyl, ferner gegebenenfalls durch Niederalkoxy, z.B. Methoxy, Amino, Niederalkanoylamino, z.B. Acetylamino, Carboxy, gegebenenfalls substituiertes Niederalkoxycarbonyl, z.B. Aethoxycarbonyl, oder 2- oder vorzugsweise 1-(Niederalkanoyloxy)-niederalkoxycarbonyl, oder Carbamoyl substituiertes Niederalkyl, z.B. Aethyl oder n-Propyl bedeutet, oder Ac ausserdem unsubstituiertes oder durch Niederalkoxy, wie Methoxy, oder Amino substituiertes Niederalkoxycarbonyl, wie Aethoxycarbonyl,

oder Carbamoyl darstellt, als Racematgemische, Racemate, optische Antipoden oder deren Salze.

Die Erfindung betrifft insbesondere pharmazeutische Präparate enthaltend Verbindungen der Formel I, worin n für 1 steht, und R Niederalkyl, z.B. Methyl, Niederalkoxyniederalkyl, z.B. 2-Methoxyäthyl, Halogenniederalkyl, z.B. Trifluormethyl, $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Sulfamoyl substituiertes Phenyl oder Pyridyl, ferner gegebenenfalls durch Niederalkoxy, z.B. Methoxy, Amino, Niederalkanoylamino, z.B. Acetylamino und Carboxy oder gegebenenfalls substituiertes Niederalkoxycarbonyl, z.B. Aethoxycarbonyl, ferner 2- oder vorzugsweise 1-(Niederalkylcarbonyloxy)-niederalkoxycarbonyl, oder Carbamoyl substituiertes Niederalkyl, z. B. Aethyl oder n-Propyl bedeutet, oder Ac ausserdem Niederalkoxycarbonyl, wie Aethoxycarbonyl, oder Carbamoyl darstellt, als Racematgemische, Racemate, optische Antipoden oder deren Salze.

Die Erfindung betrifft insbesondere pharmazeutische Präparate enthaltend Verbindungen der Formel I, worin n für 1 steht, und R Niederalkyl, z.B. Methyl, Niederalkoxyniederalkyl, z.B. 2-Methoxyäthyl, $R_1$ in einer Gruppe Ac gegebenenfalls durch Niederalkyl, z.B. Methyl, Halogenniederalkyl, z.B. Trifluormethyl, Hydroxy, Niederalkoxy, z.B. Methoxy, Halogen, Carbamoyl, Cyano und/oder Sulfamoyl substituiertes Phenyl, ferner gegebenenfalls durch Amino, Niederalkanoylamino, z.B. Acetylamino, und Carboxy oder gegebenenfalls substituiertes Niederalkoxycarbonyl, wie Niederalkoxycarbonyl z.B. Aethoxycarbonyl oder 2- oder vorzugsweise 1-(Niederalkylcarbonyloxy-niederalkoxycarbonyl, wie z.B. Pivaloyloxy-methoxycarbonyl substituiertes Niederalkyl, z.B. Aethyl, n-Propyl oder n-Butyl bedeutet, oder Ac ausserdem Niederalkoxycarbonyl, wie Aethoxycarbonyl, darstellt, als Racematgemische, Racemate, optische Antipoden oder deren Salze.

Die Erfindung betrifft insbesondere neue Verbindungen der Formel I, worin n eine ganze Zahl von 1-4 darstellt und R gegebenenfalls substituiertes Niederalkyl, Niederalkenyl oder Niederalkinyl, oder einen mono- oder bicyclischen, carbocyclischen oder heterocyclischen aromatischen Kohlenwasserstoffrest bedeutet, Ac einen Rest der Formel $-C(=O)-R_1$ darstellt, worin $R_1$ einen gegebenenfalls substituierten aliphatischen, cycloaliphatischen, aromatischen oder araliphatischen Kohlenwasserstoffrest, oder den Rest eines Monoesters oder Monoamids der Kohlensäure bedeutet, mit der Massgabe, dass R als in 3-Stellung gebundenes Niederalkyl 2-7 Kohlenstoffatome hat, wenn Ac die Acetylgruppe bedeutet, und mit der weiteren Massgabe, dass R als in 3-, 4-, 5- oder 6-Stellung gebundenes Niederalkyl 2-7 Kohlenstoffatome hat, wenn Ac die Benzoylgruppe bedeutet, als Racematgemische, Racemate, optische Antipoden oder deren Salze.

Die Erfindung betrifft insbesondere neue Verbindungen der Formel I worin n eine ganze Zahl von 1-4 darstellt und R Niederalkyl, gegebenenfalls veräthertes oder verestertes Hydroxyniederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl, wie Aminoniederalkyl, N-Niederalkylaminoniederalkyl oder N,N-Diniederalkylaminoniederalkyl, Niederalkanoylaminoniederalkyl oder Niederalkoxycarbonylaminoniederalkyl, durch gegebenenfalls verestertes oder amidiertes Carboxy, wie Carboxy, Aethoxycarbonyl oder gegebenenfalls substituiertes Carbamoyl, wie Carbamoyl, substituiertes Niederalkyl, wie Carboxyniederalkyl, Niederalkoxycarbonylniederalkyl, Carbamoylniederalkyl, Oxo-niederalkyl, ferner Cyanniederalkyl, gegebenenfalls substituiertes Arylniederalkyl, worin Aryl ein monocyclischer, aromatischer Kohlenwasserstoffrest, z.B. Phenyl, oder ein bicyclischer aromatischer Kohlenwasserstoffrest, wie 1- oder 2-Naphthyl, oder teilweise gesättigtes Naphthyl, wie 1,2,3,4-Tetrahydro-5-naphthyl ist, wobei Substituenten Niederalkyl, Hydroxy und/oder Halogen,

Hydroxyniederalkyl, Niederalkoxyniederalkyl, Halogenniederalkyl, Niederalkoxy, Niederalkenyloxy, oder gegebenenfalls niederalkyliertes oder acyliertes Aminoniederalkyl, z.B. Mono- oder Diniederalkylaminoniederalkyl oder Niederalkanoylaminoniederalkyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls acyliertes Amino, z.B. Amino, Niederalkylamino, Niederalkoxycarbonylamino, Niederalkylamino oder Diniederalkylamino sein und solche Substituenten gleich oder verschieden und 1-3-fach vorhanden sein können, bedeutet, oder R und das in der Gruppe Ac enthaltene $R_1$ jeweils für Aryl, wie Phenyl, 1- oder 2-Naphthyl oder teilweise gesättigtes Naphthyl, wie 1,2,3,4-Tetrahydro-5-naphthyl oder einen über ein Ringkohlenstoffatom mit dem Pyridylrest verbundenen, ein Sauerstoff-, Schwefel- oder Stickstoffatom und gegebenenfalls 1-3 zusätzliche Stickstoffatome als Ringglieder enthaltenden Rest, vorzugsweise mit 5 oder 6 Ringgliedern steht, der mindestens partiell hydriert und in diesem Fall durch Oxo substituiert sein kann, wobei in einem bicyclischen heterocyclischen Rest der zweite Ring ein ankondensierter Benzoring sein kann, und insbesondere Pyridyl, z.B. 2-, 3- oder 4-Pyridyl, oder Dihydro-oxo-pyridyl z.B. 1,6-Dihydro-6-oxo-2-pyridinyl, Pyridazinyl, z.B. 3-Pyridazinyl, Pyrazinyl, z.B. 2-Pyrazinyl, Pyrimidinyl, z.B. 2-, 4- oder 5-Pyrimidinyl, oder Dihydro-oxo-pyrimidinyl z.B. 3,4-Dihydro-4-oxo-2-pyrimidinyl, Furyl z.B. 2- oder 3-Furyl, Pyrryl z.B. 2- oder 3-Pyrryl, Thienyl z.B. 2- oder 3-Thienyl, Oxazolyl z.B. 2- oder 4-Oxazolyl, Thiazolyl, z.B. 2-, 4- oder 5-Thiazolyl, Thiadiazolyl z.B. 1,2,4-Thiadiazol-3- oder -5-yl oder 1,2,5-Thiadiazol-3-yl, Imidazolyl z.B. Imidazol-2- oder -4-yl, Pyrazolyl z.B. 3- oder 4-Pyrazolyl, Triazolyl, z.B. 1,2,3-Triazol-4-yl oder 1,2,4-Triazol-3-yl, Tetrazolyl wie Tetrazol-5-yl, ferner Indolyl z.B. Inol-4-yl, Chinolinyl, z.B. 2-Chinolinyl, Isochinolinyl, z.B. 1-Isochinolinyl, Benzimidazolyl, z.B. 2-Benzimidazolyl, Benzofuranyl, z.B. 4- oder 5-Benzofuranyl, Benzothienyl,

z.B. 4- oder 5-Benzothienyl, oder Naphthyridinyl z.B. 1,8-Naphthyridin-2-yl bedeutet, wobei solche Arylreste in erster Linie an einem Ringkohlenstoffatom, aber auch einen sekundären Ringstickstoffatom stehen und ein- oder mehrfach, vorzugsweise höchstens vierfach, durch gegebenenfalls substituiertes Niederalkyl, z.B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl wie Aminoniederalkyl, N-Niederalkylaminoniederalkyl, N,N-Diniederalkylaminoniederalkyl, Niederalkanoylaminoniederalkyl oder Niederalkoxycarbonylaminoniederalkyl, oder Niederalkenyl oder Niederalkinyl, gegebenenfalls veräthertes oder verestertes Hydroxy oder Mercapto, wie Hydroxy, Niederalkoxy, Phenylniederalkoxy, Halogen, Mercapto, Niederalkylthio, Phenylniederalkylthio, durch gegebenenfalls veräthertes oder verestertes Hydroxy oder Mercapto, oder durch Acyl substituiertes Niederalkoxy, z.B. Niederalkoxy, Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkinyloxy, Niederalkylthio, Acyl, z.B. Niederalkanoyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Niederalkanoylamino, Niederalkoxycarbonylamino, Niederalkylsulfonyl, Sulfamoyl, gegebenenfalls substituiertes Ureido, ferner Amino, N-Niederalkylamino oder N,N-Diniederalkylamino, substituiert sein können, das in einer Gruppe Ac enthaltene $R_1$ ausserdem gegebenenfalls durch gegebenenfalls veräthertes oder verestertes Hydroxy, wie Hydroxy, Niederalkoxy und/oder Halogen, gegebenenfalls substituiertes wie niederalkyliertes oder acyliertes Amino, wie Amino, N-Niederalkylamino oder N,N-Diniederalkylamino, Niederalkanoylamino und/oder gegebenenfalls funktionell abgewandeltes Carboxy, wie

Carboxy, verestertes Carboxy, z.B. gegebenenfalls substituiertes Niederalkoxycarbonyl, gegebenenfalls substituiertes Carbamoyl, oder Cyano, wobei substituierte Reste einen oder mehrere Substituenten, die gleich oder verschieden und 1-3-fach vorhanden sein können, in irgendwelchen der zur Substitution geeigneten Stellungen aufweisen können, substituiertes Niederalkyl, Niederalkenyl, Niederalkinyl, Phenylniederalkyl, Phenylniederalkenyl, Phenylniederalkinyl, mono- oder polycyclisches Cycloalkyl, etwa Cycloalkyl, bedeutet, oder Ac ausserdem unsubstituiertes oder substituiertes, z.B. freies oder veräthertes Hydroxy, wie Niederalkoxy, oder unsubstituiertes oder substituiertes Amino, wie Amino, Niederalkylamino, und in erster Linie disubstituiertes Amino, wie Diniederalkylamino, N-Niederalkyl-N-phenylniederalkylamino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder Halogen substituiertes Phenyl enthaltendes Niederalkoxycarbonyl, ferner N-unsubstituiertes oder N-mono- oder N,N-disubstituiertes Carbamoyl, wie Niederalkylcarbamoyl, Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylencarbamoyl darstellt, mit der Massgabe, dass R als in 3-Stellung gebundenes Niederalkyl 2-7 Kohlenstoffatome hat, und z.B. Aethyl, n-Propyl oder n-Butyl darstellt, wenn Ac die Acetylgruppe ist, und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl bedeutet, wenn Ac die Benzoylgruppe ist, als Racematgemische, Racemate, optische Antipoden oder deren Salze.

Die Erfindung betrifft insbesondere neue Verbindungen der Formel I, worin n für 1 oder 2 steht und R Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. Acetylamino-

methyl, bedeutet, und $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. 2-Acetylaminoäthyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Niederalkanoylamino, z.B. Acetylamino, oder Sulfamoyl substituiertes Phenyl oder Pyridyl, ferner gegebenenfalls durch Niederalkoxy, z.B. Methoxy, Amino, Niederalkanoylamino, z.B. Acetylamino, Carboxy, gegebenenfalls substituiertes Niederalkoxycarbonyl, z.B. Aethoxycarbonyl oder 2- oder 1-(Niederalkylcarbonyloxy)-niederalkoxycarbonyl oder Carbamoyl substituiertes Niederalkyl, z.B. Aethyl oder n-Propyl bedeutet, oder Ac ausserdem unsubstituiertes oder durch Niederalkoxy, wie Methoxy, oder Amino substituiertes Niederalkoxycarbonyl, wie Aethoxycarbonyl, oder Carbamoyl darstellt, mit der Massgabe, dass R als in 3-Stellung gebundenes Niederalkyl 2-7 Kohlenstoffatome hat, und z.B. Aethyl, n-Propyl oder n-Butyl bedeutet, wenn Ac die Acetylgruppe ist, und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl darstellt, falls Ac die Benzoylgruppe ist, als Racematgemische, Racemate, optische Antipoden oder deren Salze.

Die Erfindung betrifft insbesondere neue Verbindungen der Formel I, worin n für 1 steht, und R Niederalkyl, z.B. Methyl, Niederalkoxyniederalkyl, z.B. 2-Methoxyäthyl, Halogenniederalkyl, z.B. Trifluormethyl, $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Sulfamoyl substituiertes Phenyl oder Pyridyl, ferner gegebenenfalls durch Niederalkoxy, z.B. Methoxy, Amino, Niederalkanoyl-

BAD ORIGINAL

amino, z.B. Acetylamino und Carboxy oder gegebenenfalls substituierter Niederalkoxycarbonyl, z.B. Aethoxycarbonyl oder 2- oder vorzugsweise 1-(Niederalkoxycarbonyloxy)-niederalkoxycarbonyl, oder Carbamoyl substituiertes Niederalkyl, z.B. Aethyl oder n-Propyl, bedeutet, oder Ac ausserdem Niederalkoxycarbonyl, wie Aethoxycarbonyl oder Carbamoyl darstellt, mit der Massgabe, dass R als in 3-Stellung gebundenes Niederalkyl 2-7 Kohlenstoffatome hat, und z.B. Aethyl, n-Propyl oder n-Butyl ist, falls Ac die Acetylgruppe bedeutet, und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl bedeutet, falls Ac die Benzoylgruppe ist, als Racematgemische, Racemate, optische Antipoden oder deren Salze.

Die Erfindung betrifft insbesondere neue Verbindungen der Formel I, worin n für 1 steht, und R Niederalkyl, z.B. Methyl, Niederalkoxyniederalkyl, z.B. 2-Methoxyäthyl, $R_1$ in der Gruppe Ac gegebenenfalls durch Niederalkyl, z.B. Methyl, Halogenniederalkyl, z.B. Trifluormethyl, Hydroxy, Niederalkoxy, z.B. Methoxy, Halogen, Carbamoyl, Cyano und/oder Sulfamoyl substituiertes Phenyl, ferner gegebenenfalls durch Amino, Niederalkanoylamino, z.B. Acetylamino, und Carboxy oder gegebenenfalls substituiertes Niederalkoxycarbonyl, wie Niederalkoxycarbonyl, z.B. Aethoxycarbonyl oder 2- oder vorzugsweise 1-(Niederalkylcarbonyloxy)-niederalkoxycarbonyl substituiertes Niederalkyl, z.B. Aethyl, n-Propyl oder n-Butyl bedeutet, oder Ac ausserdem Niederalkoxycarbonyl, wie Aethoxycarbonyl, darstellt, mit der Massgabe, dass R als in 3-Stellung gebundenes Niederalkyl 2-7 Kohlenstoffatome hat, und z.B. Aethyl, n-Propyl oder n-Butyl bedeutet, wenn Ac die Acetylgruppe bedeutet, und mit der weiteren Massgabe, dass R als in 3-, 4-, 5- oder 6-Stellung gebundenes Niederalkyl 2-7 Kohlenstoffatome hat und z.B. vorgenanntes Aethyl, n-Propyl oder n-Butyl bedeutet, falls Ac die Benzoylgruppe ist, als Racematgemische, Racemate, optische Antipoden oder deren Salze.

BAD ORIGINAL

Die Erfindung betrifft insbesondere neue Verbindungen der Formel I, worin n für 1 steht und R Niederalkyl, z.B. n-Butyl, ferner Niederalkoxyniederalkyl, z.B. 3-Methoxy-n-propyl, ist und $R_1$ in der Gruppe Ac durch Amino oder insbesondere Niederalkanoylamino, z.B. Acetylamino, und durch Carboxy, oder Niederalkoxycarbonyl, z.B. Aethoxycarbonyl, ferner 2- oder vorzugsweise 1-(Niederalkylcarbonyloxy)-niederalkoxycarbonyl, z.B. Pivalyl-carbonyloxy-methoxycarbonyl, oder Carbamoyl, vorzugsweise in der ω-Stellung, substituiertes Niederalkyl, z.B. Aethyl oder n-Propyl, bedeutet, als Racematgemische, Racemate, optische Antipoden, oder deren Salze, insbesondere deren pharmazeutisch verwendbaren Salze.

Die Erfindung betrifft insbesondere neue Verbindungen der Formel I, worin n für 1 steht und R in 5-Stellung gebundenes Niederalkyl, z.B. n-Butyl, ferner Niederalkoxyniederalkyl, z.B. 3-Methoxy-n-propyl, ist, und $R_1$ in der Gruppe Ac in der ω-Stellung durch Amino oder in erster Linie durch Niederalkanoylamino, z.B. Acetylamino, und durch Carboxy oder Niederalkoxycarbonyl, z.B. Aethoxycarbonyl, ferner 1-(Niederalkylcarbonyloxy)-niederalkoxycarbonyl, z.B. Pivalyl-carbonyloxy-methoxycarbonyl, substituiertes n-Propyl ist, wobei eine Verbindung mit einer solchermassen definierten Gruppe $R_1$ vorzugsweise in optisch aktiver Form, insbesondere in der L-Form vorliegt, oder deren Salze, insbesondere deren pharmazeutisch verwendbaren Salze.

Die Erfindung betrifft insbesondere die in den Beispielen beschriebenen neuen Verbindungen der Formel I.

Die neuen Verbindungen der Formel I werden in an sich bekannter Weise hergestellt. Man kann sie erhalten, indem man z.B. eine Verbindung der Formel

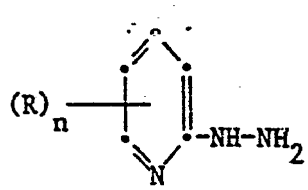

(II)

mit einer Verbindung der Formel

$$HOOC-R_1 \qquad (III),$$

oder einem reaktionsfähigen Derivat einer solchen Carbonsäure, oder mit einem reaktionsfähigen Derivat eines Monoesters oder Monoamids der Kohlensäure (IV) umsetzt, und, wenn erwünscht, eine so erhältliche Verbindung der Formel I in eine andere Verbindung der Formel I umwandelt, und/oder, wenn erwünscht, ein erhaltenes Racematgemisch in die Racemate, oder ein erhaltenes Racemat in die optischen Antipoden auftrennt, und/oder, wenn erwünscht, eine erhaltene freie Verbindung der Formel I in ein Salz oder ein erhaltenes Salz in eine freie Verbindung oder ein anderes Salz überführt.

Reaktionsfähige Derivate der durch die Formel III definierten Carbonsäuren sind z.B. Säureanhydride, beispielsweise durch intramolekularen Ringschluss von der Formel III umfassten Dicarbonsäuren gebildete Säureanhydride, z.B. entsprechend substituierte Glutarsäureanhydride, ferner gemischte Säureanhydride, z.B. solche mit Halogenwasserstoffsäuren, wie die Chloride oder Bromide, oder mit z.B. Niederalkancarbonsäuren, wie Essigsäure oder Propionsäure, Niederalkoxyalkancarbonsäuren, wie 2-Methoxyessigsäure, ferner die Azide, Cyanide oder Amide solcher Carbonsäuren. Reaktionsfähige Derivate von Carbonsäuren der Formel III sind insbesondere Ester, z.B. Niederalkyl-, wie Methyl- oder tert.-Butylester, ferner aktivierte Ester, etwa mit Cyanmethanol, N-Hydroxysuccinimid oder N-Hydroxybenztriazol, ferner mit Arylniederalkanolen, etwa gegebenenfalls durch Niederalkyl, z.B. Methyl oder Niederalkoxy z.B. Methoxy, substituiertem Benzylalkohol, oder Phenolen, die gegebenenfalls durch geeignete Substituenten aktiviert sind z.B. durch Halogen, etwa 4-Halogen, wie 4-Chlor, Niederalkoxy, etwa 4-Niederalkoxy wie 4-Methoxy, 4-Nitro- oder 2,4-Dinitro, wie etwa 4-Chlorphenol, 2,3,4,5,6-Pentachlorphenol, 4-Methoxyphenol, 4-Nitro- oder 2,4-Dinitrophenol, ferner mit Cycloalkanolen, wie etwa Cyclopentanol oder Cyclohexanol, die

BAD ORIGINAL

gegebenenfalls durch Niederalkyl, z.B. Methyl, substituiert sein können, gebildete Ester. Reaktionsfähige Derivate von Monoestern oder Monoamiden der Kohlensäure sind z.B. die Halogenide, etwa Chloride oder Bromide davon. Die Umsetzung wird in an sich bekannter Weise in Ab- oder Anwesenheit eines Lösungsmittels oder Lösungsmittgelgemisches und, wenn notwendig, unter Kühlen oder Erwärmen, in einem offenen oder geschlossenen Gefäss und/oder in einer Inertgasatmosphäre, z.B. unter Stickstoff durchgeführt. Die Umsetzung von freien Carbonsäuren der Formel III mit Verbindungen der Formel II erfolgt nötigenfalls in Gegenwart eines sauren, wasserabspaltenden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, in inerten organischen Lösungsmitteln, durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder Alkoholaten, z.B. Alkalimetall- niederalkanolaten, etwa Natriummethylat, oder auch anorganischer Basen, z.B. Alkalimetall- oder Erdalkalimetallhydroxiden oder -carbonaten, wie Natrium-, Kalium- oder Calciumhydroxid bzw. -carbonat, umgesetzt.

Die Umsetzungen mit Estern, etwa solchen der genannten Art, oder mit reaktionsfähigen Estern, z.B. Cyanmethyl- oder Pentachlorphenyl- oder N-Hydroxysuccinimidestern, werden beispielsweise in einem gegenüber den Reaktionsteilnehmern inerten Lösungsmittel bei erhöhter oder erniedrigter Temperatur durchgeführt.

Die Umsetzungen mit reaktionsfähigen Derivaten von Monoestern oder Monoamiden der Kohlensäure, etwa der Halogenide erfolgt üblicherweise in einem an der Reaktion nicht teilnehmenden Lösungsmittel, unter Kühlen oder Erwärmen, gegebenenfalls in Anwesenheit eines säurebindenden Mittels, etwa einer organischen Base, etwa einer Stickstoffphase, wie Triäthylamin, oder eines Ueberschusses an Ausgangsmaterial der Formel II, oder in Gegenwart einer anorganischen Base, wie eine Alkalimetall- oder Erdalkalimetallcarbonats oder -hydroxids, wie Natrium- oder Magnesiumcarbonat oder -hydroxid.
Diese Reaktionen werden in üblicher Weise vorgenommen.

Ausgangsstoffe der Formel II sind bekannt oder können in an sich bekannter Weise hergestellt werden. So kann man diese Verbindungen erhalten, indem man z.B. eine Verbindung der Formel

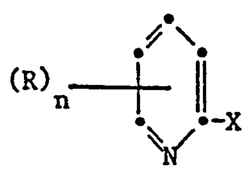

(IIa),

worin X eine geeignete Abgangsgruppe, z.B. gegebenenfalls reaktionsfähiges verestertes Hydroxy, wie Halogen, etwa Chlor oder Brom, oder eine mit einer organischen Sulfonsäure, z.B. einer aliphatischen oder aromatischen, wie einer Niederalkylsulfonsäure, z.B. Methansulfonsäure oder einer aromatischen, wie einer gegebenenfalls substituierten, wie niederalkylierten, und/oder halogenierten Arylsulfonsäure, z.B. Benzoesäure veresterte Hydroxygruppe, oder Niederalkoxy, wie Methoxy, gegebenenfalls veräthertes Mercapto, wie Mercapto oder Niederalkylthio, Niederalkylsulsulfinyl, wie Methylsulfinyl, Niederalkylsulfonyl, wie Methylsulfonyl oder den Rest $-SO_3H$ oder die Gruppe $-NH-NO_2$ bedeutet, mit wasserfreiem Hydrazin oder dessen Hydrat oder mit bis zu drei Schutzgruppen als Substituenten tragenden Hydrazin umsetzt, und anschliessend gegebenenfalls vorhandene Schutzgruppen abspaltet und durch Wasserstoff ersetzt.

Schutzgruppen sind z.B. solche, die mittels Reduktion einschliesslich Hydrogenolyse, oder mittels Solvolyse, z.B. Hydrolyse, abgespalten und durch Wasserstoff ersetzt werden, z.B. Arylniederalkylgruppen wie eine Phenylniederalkylgruppe, etwa Benzyl, die im aromatischen Teil gegebenenfalls durch Niederalkyl, wie Methyl, Halogen, wie Chlor, Niederalkoxy, wie Methoxy, und/oder Nitro substituiert sind. Die Umsetzung mit Hydrazin oder dessen Hydrat sowie die Abspaltung gegebenenfalls vorhandener Schutzgruppen erfolgt in üblicher Weise bei erhöhter oder erniedrigter Temperatur, in An- oder Abwesenheit eines weiteren säurebindenden Mittels, etwa einer organischen oder anorganischen Base, üblicherweise jedoch mittels eines Ueberschusses an Hydrazinverbindung, gegebenenfalls in Anwesenheit eines Lösungsmittels, z.B. Aethanol, im offenen oder geschlossenen Gefäss und/oder in einer Schutzgasatmosphäre, z.B. Stickstoff. Liegt die Gruppe X in einer Verbindung der Formel IIa als freie Hydroxygruppe vor, so erfolgt die Umsetzung mit einer Hydrazinverbindung unter verschärften Reaktionsbedingungen, z.B. bei erhöhter Temperatur; andererseits kann die Gegenwart eines die Wasserabspaltung begünstigenden Mittels, z.B. eines Carbodiimids, etwa N,N-Dicyclohexylcarbodiimid von Vorteil sein.

Ausgangsstoffe der Formel II können ferner erhalten werden, indem man eine Verbindung der Formel

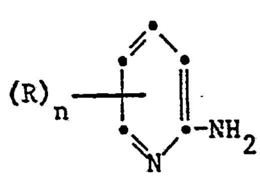
(IIb)

mit Hydroxylamin-O-sulfonsäure (IIc) umsetzt. Die Umsetzung erfolgt in üblicher Weise, bei normaler oder erhöhter Temperatur, in Ab- und Anwesenheit eines Lösungsmittels, z.B. Wasser, zweckmässigerweise in einer Schutzgasatmosphäre, etwa unter Stickstoff.

Ausgangsstoffe der Formel II können ausserdem erhalten werden, indem man in einer Verbindung der Formel

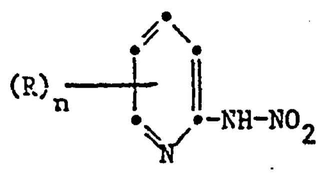

(IIc)

die Nitrogruppe zur Aminogruppe reduziert. Die Reduktion erfolgt auf übliche Weise, z.B. mittels eines geeigneten Metalls im sauren, z.B. in Gegenwart einer wässrigen Mineralsäure, wie Schwefelsäure, insbesondere aber im alkalischen Medium z.B. in einer wässrigen Lösung einer Base, wie einer anorganischen Base, z.B. Natriumhydroxid.

Ausgangsstoffe der Formel (IIc) wiederum können aus Verbindungen der vorhin erläuterten Formel (IIb) durch Nitrieren, auf übliche Weise, etwa mittels Salpetersäure in Gegenwart von konz. Schwefelsäure, erhalten werden.

Ausgangsstoffe der Formel IIa, worin R einen mono- oder bicyclischen Heteroarylrest darstellt, können erhalten werden, indem man analog wie nachfolgend bei Verbindungen der Formel VI beschrieben in einer Verbindung der Formel

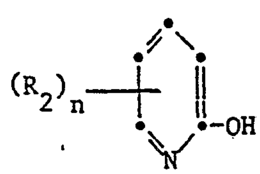

(IId),

worin $R_2$ einen zur Bildung eines mono- oder bicyclischen Heteroarylrestes R befähigten Substituenten darstellt, die mono- oder bicyclische Heteroarylgruppe R bildet, und gegebenenfalls vorhandene Schutzgruppen abspaltet und durch Wasserstoff ersetzt. So lässt sich beispielsweise eine Verbindung der Formel IId, worin $R_2$ die Formylgruppe ist, mit einer 1,2-Dicarbonylverbindung der Formel VIa

und Ammoniak in der dort beschriebenen Weise zu einer Verbindung der Formel IId umsetzen, worin anstelle der Gruppe $R_2$ ein Imidazolylrest steht, der in seiner 2-Stellung an den Pyridinring geknüpft ist.

Reaktionsfähige Derivate von Ausgangsstoffen der Formel III können auf übliche Weise erhalten werden. So ergibt die Umsetzung mit einem geeigneten halogenierend wirkenden Mittel, etwa Thionylchlorid, das entsprechende Carbonsäurechlorid, während die Veresterung mit einem Alkohol, etwa einem unsubstituierten oder substituierten Niederalkanol, oder mit N-Hydroxysuccinimid oder N-Hydroxybenztriazol oder die Umsetzung mit geeigneten Diazoverbindungen, wie unsubstituierten oder substituierten Diazoalkanen, zu entsprechenden Carbonsäureestern der Formel III führt.

Solche Verbindungen können ebenfalls erhalten werden, wenn als Ausgangsstoffe Salze, insbesondere Alkalimetall- oder Erdalkalimetallsalze der freien Carbonsäuren verwendet und diese mit reaktionsfähigen Estern von Alkoholen, wie unsubstituierten oder substituierten Niederalkanolen, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, behandelt werden, oder wenn man entsprechende hydrolysierbare Iminoester, wie entsprechende Iminoniederalkylester, zu den Estern hydrolysiert.

Der Formel III entsprechende Carbonsäureazide oder -cyanide können durch Umsetzung eines Carbonsäurehalogenids, etwa des Chlorids mit einem Salz der Stickstoffwasserstoffsäure oder der Cyanwasserstoffsäure, etwa dem Natriumsalz, zweckmässigerweise in Gegenwart eines inerten Lösungsmittels, wie Benzol, auf übliche Weise erhalten werden.

Die neuen Verbindungen der Formel I können ebenfalls erhalten werden, indem man in einer Verbindung der Formel

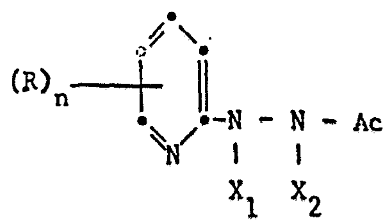

(IV),

worin $X_1$ und $X_2$ jeweils Wasserstoff oder einen durch Wasserstoff ersetzbaren Substituenten bedeuten, oder $X_1$ und $X_2$ zusammen einen zweiwertigen, durch zwei Wasserstoffatome ersetzbaren Rest bedeuten, und/oder in den Gruppen R und Ac vorhandene funktionelle Gruppen gegebenenfalls in geschützter Form vorliegen mit der Massgabe, dass mindestens einer der Reste $X_1$ und $X_2$ von Wasserstoff verschieden ist, oder mindestens $X_1$ und $X_2$ zusammen einen zweiwertigen, durch zwei Wasserstoffatome ersetzbaren Rest darstellen, oder mindestens in der Gruppe R und/oder Ac vorhandene funktionelle Gruppen in geschützter Form vorliegen, oder in einem Salz davon, die von Wasserstoff verschiedenen Gruppen $X_1$, $X_2$ oder $X_1$ und $X_2$ zusammen durch Wasserstoffatome ersetzt, und/oder in einer Gruppe R und/oder Ac die an eine oder mehrere funktionelle Gruppen gebundenen Schutzgruppen abspaltet und durch Wasserstoff ersetzt, und, wenn erwünscht, die im Anschluss an das erste Verfahren beschriebenen zusätzlichen Verfahrensschritte durchführt.

Geschützte funktionelle Gruppen in einem Rest R und/oder Ac sind z.B. geschützte Hydroxy-, Amino-, Mercapto- oder Carbonsäuregruppen, wobei letztere als Ester oder Imidoester vorliegen, deren Imidogruppe mittels Hydrolyse durch ein Sauerstoffatom ersetzt und die Estergruppe zur Carbonsäuregruppe gespalten werden kann. Die Abspaltung der Gruppen $X_1$ und $X_2$ oder $X_1$ und $X_2$ zusammen sowie in einem Rest R und/oder Ac an funktionellen Gruppen, z.B. an Hydroxy- und/oder Amino- oder Carbonsäuregruppen stehender Schutzgruppen wird mittels Solvolyse, wie Hydrolyse, Alkoholyse oder Acidolyse, oder mittels Reduktion einschliesslich Hydrogenolyse vorgenommen.

Eine besonders geeignete, abspaltbare Gruppe $X_1$ oder $X_2$, oder eine Hydroxy-, Mercapto- oder Amino-Schutzgruppe in einem Rest R und/oder Ac ist in erster Linie eine hydrogenolytisch abspaltbare α-Aryl-niederalkylgruppe, wie eine gegebenenfalls substituierte 1-Polyphenylniederalkyl- oder 1-Phenylniederalkylgruppe, z.B. Benzhydryl oder Trityl, worin Substituenten, insbesondere des Phenylteils, z.B. Niederalkyl, wie Methyl, oder Niederalkoxy wie Methoxy, sein können, und in erster Linie Benzyl. Eine Gruppe $X_1$ und $X_2$ sowie Hydroxy-Mercapto- und/oder Amino-Schutzgruppen in einem Rest R und/oder Ac können auch einen solvolytisch, wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, z.B. wie oben angegeben,und in erster Linie Trityl darstellen.

Ein durch $X_1$ und $X_2$ zusammen gebildeter abspaltbarer Rest ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl oder Niederalkoxy sein können, und insbesondere Benzyliden, oder Cycloalkyliden, z.B. Cyclopentyliden oder Cyclohexyliden.

Als Ester- oder Imidoester vorliegende geschützte Carbonsäuregruppen sind insbesondere solche, welche mittels der oben beschriebenen Methoden, insbesondere mittels Reduktion einschliesslich Hydrogenolyse in die zugrundeliegende Carbonsäure und den entsprechenden Alkohol gespalten werden. Als Esterkomponente dementsprechend geeignete Schutzgruppen kommen z.B. hydrogenolytisch abspaltbare α-Arylniederalkoxygruppen, wie eine gegebenenfalls substituierte 1-Polyphenylniederalkyl oder 1-Phenylniederalkylgruppe, z.B. Benzhydryl oder Trityl, worin Substituenten, insbesondere des Phenylteils z.B. Niederalkyl, wie Methyl, Niederalkoxy, z.B. Methoxy, und/oder Halogen sein können, und in erster Linie Benzylin Betracht.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_1$ oder $X_2$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_1$ und $X_2$ zusammen gebildete, gegebenenfalls substituierte 1-Phenylniederalkylidengruppen, sowie in einer Gruppe R und/oder Ac vorhandene Hydroxy-, Mercapto- und/oder Aminoschutzgruppen dieser Art, ferner in Carbonsäureestern oder -imidoestern als abspaltbare Gruppe vorhandenes α-Arylniederalkyl können durch Behandeln mit katalytisch aktiviertem Wasserstoff, z.B. mit Wasserstoff in Gegenwart eines Katalysators, wie eines geeigneten Edelmetallkatalysators, z.B. Palladium oder Platin abgespalten werden.

Hydrolytisch abspaltbare Gruppen $X_1$ und $X_2$, wie Acylreste von organischen Carbonsäuren, z.B. Niederalkanoyl, und von Halbestern der Kohlensäure, z.B. Niederalkoxycarbonyl, ferner z.B. Tritylreste, sowie durch die Reste $X_1$ und $X_2$ zusammen gebildete Niederalkyliden-, 1-Phenyl-niederalkyliden- oder Cycloalkylidengruppen, sowie in einem Rest R und/oder Ac an funktionellen Gruppen, wie an Hydroxy-, Mercapto- und/oder Aminogruppen stehende Schutzgruppen dieser Art können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Die Hydrolyse von Iminoestergruppen beispielsweise zu entsprechenden Resten von Monoestern oder Monoamiden der Kohlensäure erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Imidoester-salze, z.B. -hydrochloride, nach Zusatz von Wasser direkt zu den entsprechenden Estern hydrolysieren kann.

Acidolytisch abspaltbare Reste $X_1$ und $X_2$ und/oder an funktionellen Gruppen, etwa den genannten, z.B. an Hydroxy, Mercapto und/oder Amino stehende Schutzgruppen in einem Rest R und/oder Ac, sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch ein tert.-Niederalkylrest; solche Reste können z.B. durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_1$ und $X_2$ und/oder an funktionellen Gruppen, etwa den genannten, z.B. an Hydroxy, Mercapto und/oder Amino stehende Schutzgruppen in einem Rest R und/oder Ac, werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest R und/oder Ac vorhandenen funktionellen Gruppen, z.B. Hydroxy-, Mercapto- und/oder Aminogruppen stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Ausgangsstoffe der Formel IV können auf übliche Weise erhalten werden, indem man z.B. eine Verbindung der vorhin erläuterten Formel IIa mit einer Verbindung der Formel

$$\begin{array}{ccc} HN & - & N - Ac \\ | & & | \\ X_1 & & X_2 \end{array} \qquad (IVb),$$

worin $X_1$, $X_2$ und Ac obige Bedeutungen haben, umsetzt. Zweckmässigerweise arbeitet man in Gegenwart eines Kondensationsmittels, etwa für den Fall, dass X eine reaktionsfähige veresterte Hydroxygruppe ist, in Gegenwart eines säurebindenden, z.B. eines alkalischen Kondensationsmittels, etwa einer organischen Base, wie Triäthylamin, oder einer anorganischen Base, wie eines Alkalicarbonats oder -bicarbonats, wie Natriumcarbonat oder -bicarbonat. Falls X in einer Verbindung der Formel IIa die freie Hydroxygruppe darstellt, so kann die Umsetzung mit einer Verbindung der Formel IVb in Gegenwart eines wasserbindenden Mittels, etwa eines Carbodiimids, etwa N,N'-Dicyclohexylcarbodiimid oder N-Aethyl-N'-(3-dimethylaminopropyl)-carbodiimid in einem inerten Lösungsmittel, etwa Tetrahydrofuran, erfolgen.

Man kann aber auch so verfahren, dass man für die beschriebene Umsetzung anstelle einer Verbindung der Formel IVb eine Verbindung der Formel

$$\begin{array}{ccc} HN & - & NH \\ | & & | \\ X_1 & & X_2 \end{array} \qquad (IVc)$$

verwendet, und in die erhaltene Verbindung der Formel IV, die anstelle einer Gruppe Ac ein Wasserstoffatom trägt, die Gruppe Ac, etwa unter Verwendung einer entsprechenden Carbonsäure, die gegebenenfalls als reaktionsfähiges Derivat, wie Halogenid oder Anhydrid, oder als reaktionsfähiger Ester vorliegt, einführt.

Diese Umsetzungen erfolgen auf übliche Weise in Gegenwart eines geeigneten inerten Lösungsmittels in Ab- oder Anwesenheit eines Kondensationsmittels bei erhöhter oder erniedrigter Temperatur und nötigenfalls unter einer Schutzgasatmosphäre, wie Stickstoff.

Die neuen Verbindungen der Formel I können ebenfalls erhalten werden, indem man eine Verbindung der Formel

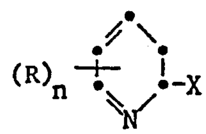

(IIa),

worin X eine geeignete Abgangsgruppe darstellt und R obige Bedeutung hat, mit einer Verbindung der Formel

$$H_2N - NH - Ac \qquad (V),$$

worin Ac obige Bedeutung hat, umsetzt, und, wenn erwünscht, die im Anschluss an das erste Verfahren beschriebenen zusätzlichen Verfahrensschritte durchführt. Eine geeignete Gruppe X ist z.B. gegebenenfalls reaktionsfähiges verestertes Hydroxy, wie Hydroxy, oder Halogen, wie Chlor oder Brom, oder eine mit einer organischen Sulfonsäure, z.B. einer aliphatischen Sulfonsäure, wie einer Niederalkansulfonsäure, wie Methansulfonsäure, oder einer aromatischen, wie einer gegebenenfalls substituierten, wie niederalkylierten und/oder halogenierten Arylsulfonsäure, etwa Benzoesäure, veresterte Hydroxygruppe, ferner Niederalkoxy, etwa Methoxy, gegebenenfalls veräthertes Mercapto, wie Mercapto oder Niederalkylthio, wie Methylthio, Niederalkylsulfinyl, wie Methylsulfinyl, Niederalkylsulfonyl, wie Methylsulfonyl, Nitro, der Rest $-SO_3H$, oder die Gruppe $-NH-NO_2$.

Die Umsetzung wird in an sich bekannter Weise, zweckmässig in einem geeigneten Lösungsmittel in Ab- oder Anwesenheit eines Kondensationsmittels, beispielsweise, wenn X eine reaktionsfähige veresterte Hydroxygruppe ist, eines säurebindenden Mittels, wie einer Base, etwa einer organischen Stickstoffbase, wie Triethylamin, oder

eines Metallalkoholats, wie eines Metall-Niederalkanolats, z.B. Natriummethylat, oder einer anorganischen Base, wie eines Alkalimetall- oder Erdalkalimetallcarbonats oder -hydroxids, wie Natrium- oder Magnesiumcarbonat, durchgeführt. Lösungsmittel können hierbei als Kondensationsmittel dienen, d.h. die Umsetzung beschleunigen. Als Lösungsmittel dieser Art, die gegebenenfalls im Gemisch mit anderen Lösungsmitteln, z.B. solchen unpolaren Charakters, verwendet werden, kommen in erster Linie solche polaren Charakters in Betracht, z.B. Niederalkanole, wie Methanol oder Aethanol, ferner Amide von Niederalkancarbonsäuren, z.B. Dimethylformamid, N-Methylacetamid, N,N-Dimethylacetamid, ferner Phosphorsäureamide, wie z.B. Hexamethylphosphorsäuretriamid, sowie polymethylierte Harnstoffe, wie N,N,N',N'-Tetramethylharnstoff, sowie Sulfolan, ausserdem gegebenenfalls niederalkyliertes, wie N-methyliertes 2-Pyrrolidon, ferner Dimethylsulfoxid, sowie Gemische solche Stoffe. Sofern in einer Verbindung der Formel IIa X für Hydroxy steht, wird die Umsetzung, falls nötig, in Gegenwart eines die Wasserabspaltung begünstigenden Mittels durchgeführt. Als solche kommen z.B. sauer reagierende Stoffe, wie z.B. Mineralsäuren, wie Chlorwasserstoff oder Schwefelsäure, ferner organische Säuren, insbesondere Sulfonsäuren, etwa Benzol- oder p-Toluolsulfonsäure in Betracht, welche in Gegenwart eines geeigneten, inerten Lösungsmittels, etwa Butanol, oder eines Aromaten, wie Benzol oder Toluol, die destillative, z.B. azeotrope, Entfernung des gebildeten Reaktionswassers beschleunigen. Die verfahrensgemässe Kondensation unter Bindung des gebildeten Wassers kann jedoch auch mittels eines Carbodiimids, z.B. N,N-Diäthyl- oder N,N-Dicyclohexylcarbodiimid in einem inerten Lösungsmittel erfolgen.

Diese Umsetzungen werden auf übliche Weise bei erhöhter oder erniedrigter Temperatur im offenen oder geschlossenen Gefäss, erforderlichenfalls unter einem Schutzgas wie Stickstoff, durchgeführt.

Die Ausgangsstoffe sind bekannt, oder können, falls sie neu sind, nach üblichen Methoden hergestellt werden. So erhält man Ausgangsstoffe der Formel V durch Umsetzung von gegebenenfalls Schutzgruppen enthaltendem Hydrazin, oder dessen Hydrat, welche z.B. mittels Reduktion und Hydrogenolyse, oder mittels Solvolyse, wie Hydrolyse, abspaltbar und durch Wasserstoff ersetzbar sind, beispielsweise α-Arylniederalkylgruppen, wie Benzyl, oder Acylgruppen, etwa Niederalkanoyl, wie Acetyl, mit einer die Einführung eines Acylrestes Ac gestattenden Verbindung, z.B. einer entsprechenden Carbonsäure oder eines reaktionsfähigen Derivats davon, etwa wie oben beschrieben, und nachfolgende Abspaltung gegebenenfalls vorhandener Schutzgruppen und deren Ersatz durch Wasserstoff.

Die neuen Verbindungen der Formel I, worin n für 1 steht und R einen mono- oder bicyclischen Heteroarylrest darstellt, können ebenfalls erhalten werden, indem man in einer Verbindung der Formel

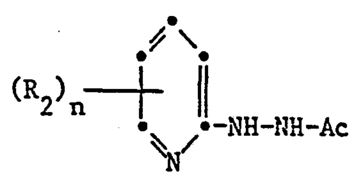

(VI),

worin Hydroxy- und/oder Aminogruppen durch Schutzgruppen geschützt sind, und $R_2$ einen zur Bildung eines mono- oder bicyclischen Heteroarylrestes R befähigten Substituenten darstellt, die mono- oder bicyclische Heteroarylgruppe R bildet, gegebenenfalls vorhandene Schutzgruppen gleichzeitig oder nachfolgend abspaltet und durch Wasserstoff ersetzt, und, wenn erwünscht, die im Anschluss an das erste Verfahren beschriebenen zusätzlichen Verfahrensschritte durchführt. Schutzgruppen sind z.B. Acyl, wie Niederalkanoyl, etwa Acetyl, die unter den zur Bildung des Heteroarylrestes R angewendeten Verfahrensbedingungen abgespalten und durch Wasserstoff ersetzt werden.

So kann man zur Herstellung einer Verbindung der Formel I, worin R einen Imidazolylrest bedeutet, der z.B. in 2-Stellung an die substituierte Pyridylgruppe, geknüpft ist, eine Verbindung der Formel VI , worin $R_2$ die Formylgruppe darstellt, in üblicher Weise mit einer 1,2-Dicarbonylverbindung der Formel

$$\begin{array}{l} R_3 - C = O \\ \quad\;\; | \\ R_4 - C = O \end{array} \qquad (VIa) ,$$

worin $R_4$ und/oder $R_3$ jeweils Wasserstoff oder einen die Heteroarylgruppe substituierenden Rest, z.B. wie oben beschrieben, darstellt in Gegenwart von Ammoniak oder mit einer der Formel VIa entsprechenden Monooximino-Verbindung umsetzen. Je nach Art des Restes $R_4$ und/oder $R_3$ kann man die Verbindung der Formel VIa als solche einsetzen oder sie kann im Reaktionsgemisch aus einer geeigneten Vorstufe in situ gebildet werden. Hierzu geeignete Vorstufen sind z.B. Derivate des Hydroxyacetons und stellen z.B. 1,1-Dihalogen-, wie z.B. 1,1-Dibromaceton dar, welches gegebenenfalls in 1 und/oder 3-Stellung durch die Gruppe $R_4$ und/oder $R_3$ substituiert ist. Weitere Vorstufen sind solche, in denen die Dicarbonylverbindung der Formel VIa in abgewandelter Form, z.B. als Acetal, etwa als Dimethylacetal, als Hydrat oder als Bisulfitadditionsverbindung, vorliegt. Aus solchen Vorstufen kann im Falle der 1,1-Dihalogenacetonderivate durch Behandlung mit hydrolysierenden Mitteln, vorzugsweise schwachen Alkalien, z.B. Natriumacetat, oder im Falle der Acetale oder Bisulfitadditionsverbindungen mit wässrigen Säuren, die Verbindung der Formel VIa in situ erhalten werden. Auf diese Weise erhält man Verbindungen der Formel I, worin der substituierte Pyridylrest eine 2-Stellung, eine Gruppe $R_4$ und/oder $R_3$ jeweils die 4- bzw. 5-Stellung des gebildeten Imidazolrestes substituieren.

Zur Herstellung von Verbindungen der Formel I, worin ein Imidazolrest R z.B. in 4-Stellung durch den substituierten Pyridylrest substituiert ist, kann man z.B. durch Umsetzung einer Verbindung der Formel VI, worin $R_2$ die Gruppe

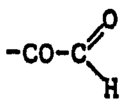 (VIb) darstellt, mit einer Verbindung der Formel

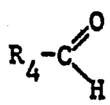 (VIc), und Ammoniak erhalten. Hierbei können die

Gruppen der Formel VIb oder VIc auch in funktionell abgewandelter Form, z.B. als Acetale, wie Dimethylacetale, als Hydrate oder als Bisulfitadditionsverbindungen vorliegen, aus denen durch Behandeln mit wässrigen Säuren die freien Verbindungen in situ gebildet und anschliessend im gleichen Reaktionsansatz weiter umgesetzt werden. Diese Umsetzungen werden in üblicher Weise, z.B. in Gegenwart eines Lösungsmittels oder Lösungsmittelgemisches, und, wenn notwendig, unter Kühlen oder Erwärmen, falls notwendig in einem geschlossenen Gefäss und/oder in einer Inertgasatmosphäre, z.B. unter Stickstoff, durchgeführt.

Die Ausgangsstoffe können in an sich bekannter Weise hergestellt werden.

So lassen sich Verbindungen der Formel VI, worin $R_2$ die Formylgruppe oder die Gruppe der Formel VIb darstellt, durch Umsetzung einer Verbindung der Formel $R_2$-Py-OH (VId), worin die dem Rest $R_2$ entsprechende Formylgruppe oder die Gruppe der Formel VIb vorzugsweise in geschützter Form, z.B. als Acetal, wie Dimethylacetal, als Hydrat oder als Bisulfitadditionsverbindung, z.B. der mit Natriumsulfit gebildeten, vorliegen, und die Hydroxygruppe auch in reaktionsfähiger, veresterter Form, z.B. als Halogen, wie Chlor oder Brom, oder als eine mit einer Sulfonsäure, wie Methan- oder einer

Aryl-, wie Benzolsulfonsäure, veresterte Hydroxygruppe vorliegt, mit einer Verbindung der oben erläuterten Formeln IVb oder V auf die dort beschriebene Weise, z.B. in Gegenwart eines Carbodiimids, wie N,N-Dicyclohexylcarbodiimid, oder falls die Hydroxygruppe reaktionsfähig verestert ist, in Gegenwart eines basischen Kondensationsmittels, und Abspaltung vorhandener Schutzgruppen, etwa wie beschrieben, herstellen.

Eine Verbindung der Formel I, worin R den Thiazolylrest bedeutet, der z.B. in 2-Stellung durch die substituierte Pyridylgruppe substituiert ist, kann erhalten werden, indem man z.B. eine Verbindung der Formel VI, worin $R_2$ die Gruppe der Formel

$$\begin{array}{l} \;\;\;\;H_2N \\ \;\;\;\;\;\;\;| \\ S = C - \end{array} \qquad (VIe)$$

bedeutet, mit einer Verbindung der Formel

$$\begin{array}{l} R_4 - CO \\ \;\;\;\;\;\;\;\;\;| \\ R_3 - CH - Hal \end{array} \qquad (VIf),$$

worin Hal für Halogen, z.B. für Chlor und insbesondere für Brom steht, und $R_4$ und/oder $R_3$ jeweils Wasserstoff oder einen die Heteroarylgruppe substituierenden Rest, z.B. wie oben beschrieben, darstellt, umsetzt. Je nach Art des Restes $R_4$ und/oder $R_3$ kann man eine Verbindung der Formel VIf in Form ihrer Derivate, z.B. der Acetale, etwa der Diäthylacetale einsetzen.

Zur Herstellung von Verbindungen der Formel I, worin R einen in 4- oder 5-Stellung durch die substituierte Pyridylgruppe substituierten Thiazolylrest darstellt, kann man z.B. eine Verbindung der Formel VI, worin $R_2$ die Gruppe der Formel

```
     -CO
      |
R3 - CH - Hal                (VIh)
```

oder der Formel

```
R4 - CO
      |
     -CH - Hal               (VIi)
```

ist, und die Carbonylgruppe gegebenenfalls in geschützter, z.B. Acetalform vorliegt, worin Hal jeweils für Halogen, z.B. für Chlor oder Brom steht, und $R_4$ und $R_3$ obige Bedeutungen haben, mit einer Verbindung der Formel

```
    H2N
      |
S = C - R4                   (VIg)
```

umsetzen. Diese Reaktionen werden in an sich bekannter Weise, z.B. in Gegenwart eines geeigneten Lösungsmittels, z.B. eines Niederalkanols, wie Aethanol, oder einer ätherartigen Flüssigkeit, wie Dioxan, gegebenenfalls bei erhöhter Temperatur und gegebenenfalls in Anwesenheit eines basischen Mittels, z.B. eines Metall-, wie Alkali- oder Erdalkalisalzes einer schwachen Säure, wie Kohlensäure oder Essigsäure, z.B. Magnesiumcarbonat oder Natriumacetat, und, falls notwendig, in einem geschlossenen Gefäss und/oder in einer Inertgasatmosphäre, z.B. unter Stickstoff durchgeführt.

Die Ausgangsstoffe lassen sich nach bekannten Methoden herstellen. So kann man eine Verbindung der Formel VI mit der Gruppe VIe als $R_2$ erhalten, indem man in einer Verbindung der Formel

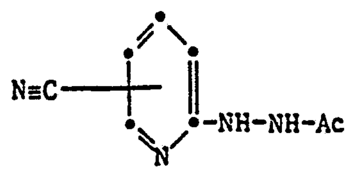

(VIk)

die Gruppe N ≡ C - durch Umsetzung mit Schwefelwasserstoff in einem geeigneten, z.B. einem basischen organischen Lösungsmittel, wie z.B. Pyridin oder Triäthylamin oder Gemischen davon, in die Gruppe VIe umwandelt.

Ausgangsstoffe der Formel VIk wiederum können erhalten werden, indem man eine Verbindung der Formel

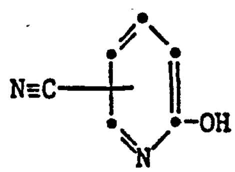

(VI 1)

worin die Hydroxygruppe gegebenenfalls in reaktionsfähiger veresterter Form, z.B. als Halogen oder eine Sulfonyloxygruppe, etwa wie oben beschrieben, vorliegt, mit einer Verbindung der Formel IVb oder V, etwa wie dort beschrieben, umsetzt, und gegebenenfalls vorhandene Schutzgruppen abspaltet und durch Wasserstoff ersetzt.

Ausgangsstoffe der Formel VI, worin $R_2$ für eine Gruppe der Formel VIh oder VIi steht, können z.B. durch Umsetzung einer Verbindung der Formel

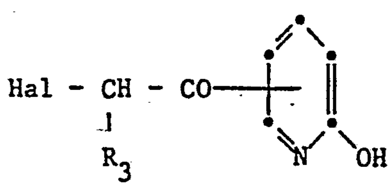

(VIm)

oder der Formel

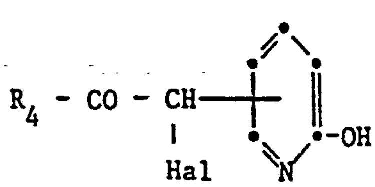

(VIn),

worin Hal jeweils für Halogen, z.B. Chlor oder Brom steht, und die Hydroxygruppe auch in reaktionsfähiger Form, z.B. wie beschrieben, etwa als Halogen, wie Chlor oder Brom vorliegen kann, mit einer Verbindung der Formel IVb oder V, etwa wie beschrieben, umsetzt, und gegebenenfalls vorhandene Schutzgruppen abspaltet und durch Wasserstoff ersetzt.

Eine Verbindung der Formel I, worin R einen Pyrimidinylrest bedeutet, der z.B. in 4(6)-Stellung an die substituierte Pyridylgruppe geknüpft ist, kann erhalten werden, indem man z.B. eine Verbindung der Formel VI, worin $R_2$ die Gruppe der Formel

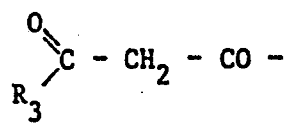 (VIo)

darstellt, worin die Carbonylgruppe(n) gegebenenfalls in geschützter, z.B. acetalisierter Form, z.B. als Dimethylacetal, oder als Bisulfitadditionsverbindung, z.B. mit Natriumbisulfit, vorliegen, mit einer Verbindung der Formel

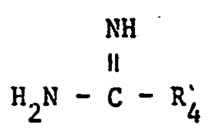 (VIp) ,

worin $R_4$ und $R_3$ obige Bedeutungen haben, umsetzt.

Diese Reaktion wird in üblicher Weise und vorzugsweise unter sauren Bedingungen, z.B. in Gegenwart eines sauer reagierenden Mittels, wie z.B. Ammoniumchlorid, vorgenommen.

Die Ausgangsstoffe können nach an sich bekannten Methoden erhalten werden.

So kann man z.B. ein Ausgangsmaterial der Formel VI mit der Gruppe VIo als $R_2$ herstellen, indem man eine Verbindung der Formel

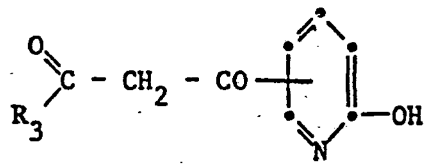

(VIq)

mit vorzugsweise geschützter, z.B. wie beschrieben, etwa acetalisierter Carboxaldehydgruppe mit einer Verbindung der Formel IVb oder V, etwa wie beschrieben, umsetzt, und gegebenenfalls vorhandene Schutzgruppen abspaltet.

Zur Herstellung von Verbindungen der Formel I, worin R einen Pyrimidinylrest darstellt, der z.B. in 2-Stellung an die substituierte Pyridylgruppe geknüpft ist, kann man z.B. eine Verbindung der Formel VI, worin $R_2$ die Gruppe der Formel

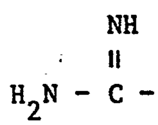

(VIr)

bedeutet, mit einer Verbindung der Formel

$$R_4 - CO - CH_2 - CO - R_3 \qquad (VIs),$$

worin die Carbonylgruppe(n) gegebenenfalls in geschützter, z.B. acetalisierter, Form, etwa sls Dimethylacetale, vorliegen und $R_4$ und $R_3$ die gegebenen Bedeutungen haben, umsetzen. Diese Umsetzung wird auf an sich bekannte Weise, z.B. unter sauren Bedingungen, etwa wie beschrieben, z.B. in Gegenwart von Ammoniumchlorid, vorgenommen.

Die Ausgangsstoffe können nach an sich bekannten Methoden hergestellt werden. So kann man z.B. eine Verbindung der Formel VI mit

der Gruppe VIr als $R_2$ erhalten, indem man z.B. eine Verbindung der oben erläuterten Formel(VIk) in den entsprechenden Iminoester, z.B. durch Sättigen einer absolut-äthanolischen Lösung einer solchen Verbindung mit Chlorwasserstoff, umwandelt und hieraus durch Umsetzung mit wasserfreiem Ammoniak eine die Gruppe (VIr) als $R_2$ enthaltende Verbindung der Formel VI, vorzugsweise in Form eines Salzes, etwa mit einer starken Säure, z.B. als Hydrochlorid, erhält, und gegebenenfalls vorhandene Schutzgruppen gleichzeitig oder nachfolgend entfernt und durch Wasserstoff ersetzt.

Bei der Auswahl des geeigneten obigen Verfahrens zur Herstellung von Verbindungen der Formel I muss darauf geachtet werden, dass vorhandene Substituenten nicht umgewandelt oder abgespalten werden, falls solche Umwandlungen bzw. Abspaltungen nicht erwünscht sind. So können insbesondere funktionell abgewandelte Carboxylgruppen, wie veresterte oder amidierte Carboxylgruppen, sowie Cyangruppen, während Solvolysen, insbesondere Hydrolysen, ferner auch bei Reduktionen an der Reaktion beteiligt sein und umgewandelt werden. Andererseits können gleichzeitige Umwandlungen von Substituenten erwünscht sein; z.B. können ungesättigte Substituenten, wie Niederalkenyl, unter den Bedingungen eines erfindungsgemäss eingesetzten Reduktionsverfahrens, z.B. zu Niederalkyl, reduziert werden.

Verfahrensgemäss erhaltene Verbindungen kann man im Rahmen der Definition der Verbindungen der Formel I in üblicher Weise in andere Endstoffe überführen, z.B. indem man geeignete Substituenten abwandelt, einführt oder abspaltet.

Freie Carboxylgruppen lassen sich in üblicher Weise verestern, beispielsweise durch Umsetzen mit einem entsprechenden Alkohol, vorteilhaft in Gegenwart einer Säure, wie einer Mineralsäure, z.B. Schwefelsäure oder Chlorwasserstoffsäure, oder in Gegenwart eines wasserbindenden Mittels, wie Dicyclohexylcarbodiimid, oder durch Umsetzen mit einer entsprechenden Diazoverbindung, z.B.

Diazomethan. Die Veresterung kann auch durch Umsetzen eines Salzes, vorzugsweise eines Alkalimetallsalzes der Säure mit einem reaktionsfähigen veresterten Alkohol, z.B. einem entsprechenden Halogenid, wie Chlorid, durchgeführt werden.

Freie Carboxylgruppen lassen sich in üblicher Weise amidieren beispielsweise durch Umsetzen mit Ammoniak, oder mit einem primären oder sekundären Amin, vorteilhaft in Gegenwart eines wasserbindenden Mittels, wie Dicyclohexylcarbodiimid, oder durch Ueberführen der Carboxylgruppe in eine Halogencarbonyl-, z.B. Chlorcarbonylgruppe und anschliessendem Umsetzen mit Ammoniak oder mit einem primären oder sekundären Amin.

In Verbindungen die eine veresterte Carboxylgruppe enthalten, kann diese in üblicher Weise, z.B. durch Hydrolyse, vorzugsweise in Gegenwart von starken Basen, wie einem Alkalimetallhydroxyd, z.B. Natrium- oder Kaliumhydroxyd, oder starken Säuren, z.B. einer starken Mineralsäure, wie einer Halogenwasserstoffsäure, z.B. Chlorwasserstoffsäure oder Schwefelsäure, in eine freie Carboxylgruppe übergeführt werden.

In Verbindungen, die eine veresterte Carboxylgruppe enthalten, kann diese in Gegenwart eines anderen Alkohols, vozugsweise im Ueberschuss, in eine den anderen Alkohol als Esterkomponente enthaltende Carboxylgruppe umgewandelt werden, z.B. in Gegenwart eines basischen Katalysators, z.B. einer Alkalimetallverbindung des zur Umesterung verwendeten Alkohols, oder eines geeigneten Katalysators, z.B. eines Edelmetallkatalysators, wie etwa eines Palladium-auf-Kohle-Katalysators.

In Verbindungen mit einer veresterten Carboxylgruppe als Substituent kann diese in üblicher Weise, z.B. durch Ammonolyse oder Aminolyse mit Ammoniak oder einem primären oder sekundären Amin in die entsprechende Carbamoylgruppe übergeführt werden.

Verbindungen mit einer unsubstituierten Carbamoylgruppe können in

üblicher Weise, z.B. durch Einwirkung wasserentziehender Mittel, wie Phosphorpentoxid, Phosphoroxychlorid oder Trifluoressigsäureanhydrid, vorzugsweise bei höheren Temperaturen, zu den entsprechenden Cyanverbindungen dehydratisiert werden.

In Verbindungen mit einer veresterten Carboxylgruppe als Substituent kann die veresterte Carboxylgruppe in üblicher Weise, z.B. durch Einwirkung einer organischen, etwa einer Diniederalkylaluminiumamid-Verbindung, z.B. Diäthylaluminiumamid, in eine Cyanogruppe umgewandelt werden.

Verbindungen, die einen Cyansubstituenten enthalten, können in üblicher Weise, z.B. in Gegenwart konzentrierter wässriger Mineralsäuren oder Alkalimetallhydroxiden, zu den entsprechenden Carbamoyl- oder direkt zu den Carboxylverbindungen hydrolysiert werden.

Verbindungen mit einer Cyangruppe als Substituenten können in üblicher Weise, z.B. durch Addition von Alkoholen in Gegenwart einer wasserfreien Säure, wie Chlorwasserstoff, und nachträglicher Hydrolyse des entstandenen Imidoesters zu den entsprechenden Verbindungen mit veresterten Carboxylgruppen alkoholysiert werden.

Verbindungen, die eine Aminogruppe als Substituenten enthalten, können in üblicher Weise, in entsprechende Acylaminoverbindungen, wie etwa Niederalkanoylaminoverbindungen, z.B. durch Umsetzung einer entsprechenden Carbonsäure, etwa einer Niederalkancarbonsäure oder einem reaktionsfähigen Derivat davon, etwa einem Halogenid, oder einem Anhydrid oder einem Ester, wie Niederalkylester, umgewandelt werden.

Verbindungen, die eine Aminogruppe als Substituenten enthalten, können in üblicher Weise in entsprechende Niederalkylamino- bzw. Diniederalkylaminoverbindungen umgewandelt werden, beispielsweise durch Umsetzung mit einem Niederalkylhalogenid, wie einem Bromid

oder Iodid davon.

Verbindungen, die in einem aromatischen Ring eine Niederalkylthio-, z.B. Methylthiogruppe, tragen, können durch Behandeln mit geeigneten desulfurierend wirkenden Mitteln, z.B. Raney-Nickel, in einem geeigneten Lösungsmittel, z.B. Dioxan, in die schwefelfreien Verbindungen umgewandelt werden.

Wie bei den Herstellungsverfahren muss auch bei der Durchführung der Zusatzschritte darauf geachtet werden, dass unerwünschte Nebenreaktionen, welche die Umwandlung zusätzlicher Gruppierungen zur Folge haben können, nicht eintreten.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäss unter Druck und/oder in einer Inertgasatmosphäre.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freie Verbindung überführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren, nicht-toxischen Salzen eignen.

Salze der neuen Verbindungen mit Basen können in an sich bekannter Weise, z.B. durch Behandeln mit sauren Mitteln, etwa verdünnten Mineralsäuren, wie Salzsäure oder Schwefelsäure, oder mit organischen Säuren, wie Essigsäure, oder mit sauren Ionenaustauschern in die freien Carbonsäuren überführt werden. Diese wiederum können durch Umsetzung mit Basen, z.B. anorganischen oder organischen Basen, entsprechende Salze bilden, wobei insbesondere pharmazeutisch annehmbare, nicht-toxische Basen, beispielsweise die genannten, verwendet werden.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als bestimmte optische Antipoden eingesetzt werden.

Erhaltene Racematgemische können auf Grund der physikalischchemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, wie Säuren oder Basen, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche optisch aktive Säuren sind z.B. die D- und L-Formen von Weinsäure, Di-O,O'-(p-Toluoyl)-weinsäure, Aepfelsäure, Mandelsäure, Camphersulfonsäure, Glutaminsäure, Asparaginsäure oder Chinasäure. Besonders gebräuchliche optisch aktive Basen sind z.B. optisch aktive Amine, wie optisch aktive Aminosäureester, (-)-Brucin, (+)-Chinidin, (-)-Chinin, (+)-Chinchonin, (+)-Dehydroabietylamin, (+)- oder (-)-Ephedrin. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z.B. analog wie in den Beispielen, beschrieben, erhalten werden. Neue Ausgangsstoffe, insbesondere solche der vorhin erläuterten Formel II, worin R und n die unter der Formel I angegebenen Bedeutungen haben, n insbesondere für 1 steht und R in 3- oder 6-Stellung, insbesondere jedoch in 5-Stellung steht, und, Niederalkoxyniederalkyl, z.B. 2-Methoxyäthyl, oder Phenylniederalkyl, z.B. Benzyl bedeutet, oder falls n 1 ist, Niederalkyl mit 2-7 Kohlenstoffatomen, wie Aethyl, n-Propyl oder n-Butyl darstellt und mit der weiteren Massgabe, dass R als Niederalkyl in 4-Stellung 3-7 Kohlenstoffatomen hat und z.B. n-Propyl oder n-Butyl bedeutet, wenn n 1 ist, oder deren Salze, insbesondere pharmazeutisch verwendbare, nicht-toxische Säureadditionssalze, sowie Verfahren zu deren Herstellung bilden ebenfalls einen Gegenstand der Erfindung. Die Erfindung betrifft auch verfahrensgemäss erhältliche neue Zwischenprodukte, sowie Verfahren zu deren Herstellung.

Die Erfindung betrifft ebenfalls die Verwendung der Verbindungen der Formel I oder von pharmazeutisch verwendbaren Salzen von solchen Verbindungen, insbesondere als pharmakologisch, insbesondere im renalen und cardiovasculären Bereich, aktive Verbindungen. Dabei kann man sie, vorzugsweise in Form von pharmazeutischen Präparaten, in einem Verfahren zur prophylaktischen und/oder therapeutischen Behandlung der Hypertonie verwenden. Die Dosierung des Wirkstoffs, der allein oder zusammen mit dem üblichen Träger- und Hilfsmaterial verabreicht, wird, hängt von der zu behandelnden Spezies, deren Alter und individuellen Zustand, sowie der Verabreichungsweise ab. Die tägliche in einer oder mehreren, vorzugsweise höchstens 4 Einzeldosen zu verabreichende Gesamtdosis der antihypertensiv wirkenden Verbindungen liegen für Mammalien mit einem Körpergewicht von etwa 70 kg, je nach Art der Erkrankung, individuellem Zustand und Alter, vorzugsweise zwischen 150 und 750 mg.

Die Erfindung betrifft weiterhin pharmazeutische Präparate, welche eine pharmakologisch wirksame Menge der Aktivsubstanz, gegebenenfalls zusammen mit pharmazeutisch verwendbaren Trägerstoffen enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder Calciumstearat, und/oder Polyäthylenglykol,

aufweisen. Tabletten können ebenfalls Bindemittel, z.B. Magnesiumaluminiumsilikat, Stärken, wie Mais-, Weizen-, Reis- oder Pfeilwurzelstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die folgenden Beispiele dienen zur Illustration der Erfindung, Temperaturen werden in Celsiusgraden angegeben.

Beispiel 1: Eine Lösung von 15,1 g N-(Benzyloxycarbonyl)-L-glutaminsäure-5-[$N^2$-(5-n-butyl-2-pyridyl)-hydrazid]-1-benzylester in 150 ml Methanol wird nach Zusatz von 1,5 g Palladium-auf-Kohle-Katalysator (5%) unter Normalbedingungen bis zur beendeten Wasserstoffaufnahme hydriert. Die Suspension wird mit Methanol verdünnt, erwärmt und durch ein Filterhilfsmittel filtriert. Das Filtrat wird am Rotationsverdampfer auf ein kleines Volumen eingeengt, mit Isopropanol verrührt und anschliessend filtriert. Durch Umkristallisation des Rückstands aus Methanol und Trocknen im Hochvakuum erhält man das L-Glutaminsäure-5-[$N^2$-(5-n-butyl-2-pyridyl)-hydrazid]-hemihydrat vom Smp. 155-157°;

$[\alpha]_D^{20}$ : + 27° ± 1° (c = 0,5 - 1% in Methanol).

Das Ausgangsmaterial kann wie folgt hergestellt werden:

Eine Lösung von 6,9 g 2-Hydrazino-5-n-butyl-pyridin in 175 ml trockenem Tetrahydrofuran wird im Eisbad gekühlt, unter Rühren portionenweise mit 19,6 g N-(Benzyloxycarbonyl)-L-glutaminsäure-1-benzylester-5-(N-hydroxysuccinimid)-ester versetzt, und anschliessend noch 2 Stunden bei 0° gerührt. Nach dem Entfernen des Lösungsmittels am Rotationsverdampfer und Chromatographieren des Rückstands über Kieselgel unter Verwendung von Essigsäureäthylester als Laufmittel erhält man aus den Hauptfraktionen den N-(Benzyloxycarbonyl)-L-glutaminsäure-5-[$N^2$-(5-n-butyl-2-pyridyl)-hydrazid]-1-benzylester als Oel, welches als solches weiterverarbeitet wird.

<u>Beispiel 2:</u> Zu einer Suspension von 1,47 g L-Glutaminsäure-5-[$N^2$-5-n-butyl-2-pyridyl)-hydrazid und 1,33 g wasserfreiem Natriumcarbonat in 20 ml Dimethylformamid wird unter Rühren bei Raumtemperatur eine Lösung von 0,53 g Essigsäureanhydrid in 5 ml Dimethylformamid im Verlaufe von 15 Minuten zugetropft. Es wird weitere 15 Stunden bei Raumtemperatur gerührt, anschliessend durch ein Filterhilfsmittel filtriert und das Filtrat am Rotationsverdampfer vom Lösungsmittel befreit. Der zurückbleibende gelbliche Schaum wird an Kieselgel mit einem Lösungsmittelgemisch Chloroform/Methanol/Ammoniak 5:3:1 chromatographiert. Die Hauptfraktionen werden vereinigt und am Rotationsverdampfer vom Lösungsmittel befreit. Zur Herstellung des Ammoniumsalzes wird der Rückstand in einem Ueberschuss an methanolischem Ammoniak gelöst, die Lösung eingedampft und das Produkt aus Isopropanol umkristallisiert, wobei man das Ammoniumsalz vom N-Acetyl-L-glutaminsäure-5-[$N^2$-(5-n-butyl-2-pyridyl)-hydrazid] enthält; Smp. 147 - 149°; $[\alpha]_D^{20}$: +29° ± 1°, (c = 0,5-1 % in Methanol); Rf-Wert im System Methylenchlorid/Methanol/$NH_4OH$ konz. (5:3:1) 0,76.

<u>Beispiel 3:</u> Eine Lösung von 2,7 g [N-Acetyl-L-glutaminsäure-5-[$N^2$-(5-n-butyl-2-pyridyl)-hydrazid]-1-benzylester] in 50 ml Methanol wird nach Zusatz von 0,5 g Palladium-auf-Kohle-Katalysator (5%) unter Normalbedingungen bis zur beendeten Wasserstoffaufnahme hydriert.

Es wird vom Katalysator abfiltriert und das Filtrat am Rotationsverdampfer vom Lösungsmittel befreit. Der Rückstand wird an Kieselgel mit einem Lösungsmittelgemisch Chloroform/Methanol/Ammoniak 5:3:1 chromatographiert. Die Hauptfraktionen werden vereinigt und vom Lösungsmittel befreit. Zur Herstellung des Ammoniumsalzes wird der Rückstand in einem Ueberschuss von methanolischem Ammoniak gelöst, die Lösung erneut eingedampft und das Produkt aus Isopropanol umkristallisiert, wobei man das Ammoniumsalz vom N-Acetyl-L-glutaminsäure-5-[$N^2$-(5-n-butyl-2-pyridyl)-hydrazid] erhält; Smp. 147-149°; $[\alpha]_D^{20}$ : + 29° ± 1°, (c = 0,5 - 1% in Methanol).

Das Ausgangsmaterial kann wie folgt hergestellt werden:

Eine Suspension von 3,17 g 2-Hydrazino-5-n-butyl-pyridin-dihydrochlorid in 60 ml trockenem Tetrahydrofuran wird auf 5° abgekühlt und unter Rühren zuerst mit 4,2 ml Triäthylamin und nach 5 Minuten mit 5,0 g N-Acetyl-L-glutaminsäure-1-benzylester-5-(N-hydroxysuccinimid)-ester versetzt. Anschliessend wird das Gemisch während 15 Stunden bei Raumtemperatur gerührt, vom Triäthylamin-hydrochlorid abfiltriert und das Filtrat am Rotationsverdampfer bei einer Badtemperatur von 30-40° vom Lösungsmittel befreit. Der leicht grünliche Rückstand wird über Kieselgel mit einem Chloroform-Methanol-Gemisch (95:5) chromatographiert.

Der aus den Hauptfraktionen in Form eines farblosen Schaums erhaltene

N-Acetyl-L-glutaminsäure-5-[$N^2$-5-n-butyl-2-pyridyl)-hydrazid]-1-benzylester wird als solcher weiterverarbeitet; Rf-Wert im System Methylenchlorid/Methanol (9:1) auf Kieselgel 0,29.

Beispiel 4: Eine Lösung von 7,0 g 2-Hydrazino-5-n-butyl-pyridin in 210 ml trockenem Tetrahydrofuran wird mit 5,9 ml Triäthylamin versetzt, anschliessend bei Raumtemperatur unter Rühren im Verlaufe einer Stunde eine Lösung von 10,8 g 3-Sulfamoyl-4-chlor-benzoylchlorid in 120 ml Tetrahydrofuran zugetropft und dann weitere $2\frac{1}{2}$ Stunden bei dieser Temperatur gerührt. Es wird von Triäthylaminhydrochlorid abfiltriert, und das Filtrat unter Rühren mit 300 ml wässriger 2-n.-Essigsäure versetzt. Das sich bildende Kristallisat wird abfiltriert, getrocknet und 2 mal aus Essigsäureaethylester umkristallisiert, wobei man bei der letzten Umkristallisation dem Lösungsmittel 2,5 ml Eisessig zusetzt.
Nach dem Abfiltrieren und Trocknen des Niederschlags erhält man das $N^2$-(5-n-Butyl-2-pyridyl)-3-sulfamoyl-4-chlor-benzoesäurehydrazidacetat vom Smp. 142-144°.

Beispiel 5: Zur Suspension von 3,2 g Natriumhydrid in 40 ml Toluol wird im Laufe einer Viertelstunde eine Lösung von 10,0 g 2-Hydrazino-5-n-butylpyridin in 150 ml Toluol und anschliessend innerhalb von 10 Minuten eine Lösung von 9,2 g Picolinsäureäthylester in 20 ml Toluol gegeben. Man erhitzt darauf vorsichtig im Laufe einer halben Stunde auf 40° (zunächst exotherme Reaktion), dann noch weitere $2\frac{1}{2}$ Stunden unter Rückfluss zum Sieden. Nach dem Erkalten extrahiert man das Reaktionsgemisch mit insgesamt 200 ml 2-n.Salzsäure, wäscht die wässrig-saure Phase mit Aether, stellt die wässrig-saure Phase mittels Natronlauge alkalisch ein und extrahiert erschöpfend mit Aether. Nach dem Trocknen der Aetherlösung versetzt man diese mit einer 5-n.Lösung von Chlorwasserstoff in Aether bis zur stark sauren Reaktion, filtriert den Niederschlag ab und kristallisiert aus Aethanol um, wonach man das $N^2$-(5-n-Butyl-2-pyridyl)-picolinsäurehydrazid-dihydrochlorid als leicht gelbstichige Kristalle vom Smp. 186-191° (Zersetzung) erhält.

Beispiel 6. Zu einer Lösung von 8,55 g 2-Hydrazino-5-n-butylpyridin in 100 ml Toluol fügt man im Verlaufe einer Viertelstunde eine Lösung von 6,5 g Chlorameisensäureäthylester in 50 ml Toluol unter Kühlung, Rühren und Durchleiten eines Stickstoffstroms. Das Reaktionsprodukt scheidet sich in öliger Form als untere Schicht ab. Zur Vervollständigung der Umsetzung erhitzt man das Gemisch noch weitere 2 Stunden unter Rückfluss, dampft anschliessend zur Trockene ein, nimmt den harzigen Rückstand in einem Gemisch von 250 ml Wasser und 10 ml 4-n.Salzsäure auf und wäscht die trübe Lösung mit 100 ml Aether. Aus der wässerigen Phase wird das Reaktionsprodukt durch Alkalisieren mit 25 ml 4-n.Sodalösung als Base freigesetzt, mit 100 ml Aether extrahiert und nach dem Trocknen der Aetherlösung über Natriumsulfat und Abdampfen zur Trockene als Rohprodukt isoliert. Man löst dieses in einem Gemisch von 100 ml Aether und 50 ml wasserfreiem Aethanol und fügt 10 ml einer 5-n.Lösung von Chlorwasserstoff in Aether zu, wonach man das N-Aethoxycarbonyl-$N^2$-(5-n-butyl-2-pyridyl)-hydrazin als Monohydrochlorid als farblose Kristalle vom Smp. 133-135° erhält, (aus Aethanol-Aether).

Beispiel 7: Zu einer Lösung von 4,0 g 2-Hydrazino-5-n.-butylpyridin in 40 ml Aether fügt man im Laufe einer halben Stunde eine Lösung von 2,5 g Essigsäureanhydrid in 20 ml Aether unter Kühlen und kräftigem Rühren zu. Nach 3-stündigem Stehenlassen lässt man unter Rühren und Kühlen eine Lösung von 7,3 ml einer 5-n.Lösung von Chlorwasserstoff in Aether, die mit 50 ml Aether verdünnt ist, zutropfen, saugt den ausgefallenen Niederschlag ab und kristallisiert aus Aethanol-Aether um. Man erhält das $N^2$-(5-n-Butyl-2-pyridyl)-N-essigsäurehydrazid als Monohydrochlorid in Form schwach gefärbter Kristalle vom Smp. 211-212°.

BAD ORIGINAL

<u>Beispiel 8:</u> Zu einer Suspension von 3,2 g Natriumhydrid in [illegible] Toluol fügt man im Verlaufe einer halben Stunde eine Lösung von 10,0 g 2-Hydrazino-5-n-butylpyridin in 150 ml Toluol und anschliessend innerhalb 10 Minuten eine Lösung von 11,7 g Fusarinsäureäthylester in 20 ml Toluol zu, erhitzt das Gemisch im Laufe einer Stunde auf 60° und anschliessend während 2 Stunden unter Rückfluss zum Sieden. Nach dem Erkalten extrahiert man das Reaktionsgemisch mit insgesamt 200 ml 2-n.Salzsäure, wäscht die wässrig-saure Phase mit Aether, stellt die wässrige Phase mit konz. Natronlauge alkalisch ein, extrahiert erschöpfend mit Aether, trocknet die Aetherlösung über Natriumsulfat und dampft zur Trockne ab. Den Rückstand löst man in 200 ml wasserfreiem Aether und versetzt die Lösung mit 20 ml einer 5-n.Lösung von Chlorwasserstoff in Aether, saugt den ausgefallenen Niederschlag ab und kristallisiert diesen aus Aethanol um. Man erhält $N^2$-(5-n-Butyl-2-pyridyl)-N-fusarinsäurehydrazid als Dihydrochlorid in Form leicht gelbstichiger Nadeln vom Smp. 175° (Zersetzung).

<u>Beispiel 9:</u> Eine Lösung von 11,5 g des nach Beispiel 3 erhaltenen N-Acetyl-L-glutaminsäure-5-[$N^2$-(5-n-butyl-2-pyridyl)-hydrazid]-1-benzylester in 120 ml wasserfreiem Methanol wird in Gegenwart von 2,3 g Palladium-auf-Kohle-Katalysator (5 %) während 24 Stunden bei Raumtemperatur gerührt. Nach dem Abfiltrieren des Katalysators wird das Filtrat am Rotationsverdampfer eingeengt, und der Rückstand über Kieselgel (FLUKA 60) unter Verwendung eines Chloroform-Methanol-Gemischs (95:5) chromatographiert. Hierbei erhält man den N-Acetyl-L-glutaminsäure-5-[$N^2$-(5-n-butyl-2-pyridyl)-hydrazid]-1-methylester, dessen Rf-Wert im System Methylenchlorid/Methanol (9:1) auf Kieselgel 0,23 ist.

<u>Beispiel 10:</u> Analog, wie in der allgemeinen Beschreibung erläutert und in den Beispielen 1 - 9 beschrieben, können unter Verwendung geeigneter Ausgangsstoffe und Reaktionsbedingungen auch folgende Verbindungen der Formel I oder deren Salze hergestellt werden:

BAD ORIGINAL

a) N-Acetyl-L-glutaminsäure-5-[$N^2$-(5-n-butyl-2-pyridyl)-hydrazid]-1-äthylester durch Umesterung des nach Beispiel 3 erhaltenen N-Acetyl-L-glutaminsäure-5-[$N^2$-(5-n-butyl-2-pyridyl)-hydrazid]-1-benzylesters mit überschüssigem wasserfreiem Aethanol in Gegenwart eines Palladium-auf-Kohle-Katalysators (5 %) bei Raumtemperatur, Aufarbeiten und Chromatographie des nach dem Abdampfen des überschüssigen Aethanols erhaltenen Rückstands über Kieselgel (FLUKA 60) wie in Beispiel 9 beschrieben; Rf-Wert im System Methylenchlorid/Methanol (9:1) auf Kieselgel 0,25.

b) N-Acetyl-L-glutaminsäure-5-[$N^2$-(5-n-butyl-2-pyridyl)-hydrazid]-1-(2,2-dimethylpropyl)-ester durch Umesterung des nach Beispiel 3 erhaltenen N-Acetyl-L-glutaminsäure-5-[$N^2$-(5-n-butyl-2-pyridyl)-hydrazid]-1-benzylesters mit überschüssigem wasserfreiem 2,2-Dimethylpropanol in Gegenwart eines Palladium-auf-Kohle-Katalysators (5 %), bei Raumtemperatur, Aufarbeiten und Chromatographie des nach dem Abdampfen des überschüssigen 2,2-Dimethylpropanols erhaltenen Rückstands über Kieselgel (FLUKA 60) wie im Beispiel 9 beschrieben; Rf-Wert im System Methylenchlorid/Methanol (9:1) auf Kieselgel 0,28.

c) N-Acetyl-L-glutaminsäure-5-[$N^2$-(5-n-butyl-2-pyridyl)-hydrazid]-1-n-butylester durch Umesterung des nach Beispiel 3 erhaltenen N-Acetyl-L-glutaminsäure-5-[$N^2$-(5-n-butyl-2-pyridyl)-hydrazid]-1-benzylesters mit überschüssigem wasserfreiem n-Butanol in Gegenwart eines Palladium-auf-Kohle-Katalysators bei einer Temperatur von 40-50°, Abfiltrieren des Katalysators, Einengen des Filtrats am Rotationsverdampfer und Chromatographie des Rückstands über Kieselgel (FLUKA 60), wie im Beispiel 9 beschrieben; Rf-Wert im System Methylenchlorid/Methanol (9:1) auf Kieselgel 0,27.

d) N-Acetyl-L-glutaminsäure-5-[$N^2$-(5-n-butyl-2-pyridyl)-hydrazid]-1-amid durch Umsetzung des nach Beispiel 1 erhaltenen L-Glutaminsäure-5-[$N^2$-(5-n-butyl-2-pyridyl)-hydrazid]-hemihydrat mit überschüssigem Methanol in Gegenwart von Cyclohexylcarbodiimid bei Raumtemperatur zum L-Glutaminsäure-5-[$N^2$-(5-n-butyl-2-pyridyl)-hydrazid]-1-methylester, dessen anschliessende Umsetzung mit überschüssigem in Methanol gelöstem Ammoniak im geschlossenen Gefäss während

5 Stunden bei Raumtemperatur und Aufarbeiten des Reaktionsgemisches zum L-Glutaminsäure-5-[$N^2$-(5-n-butyl-2-pyridyl)-hydrazid]-hemihydrat und dessen anschliessende Umsetzung mit Essigsäureanhydrid in Dimethylformamid in Gegenwart von wasserfreiem Natriumcarbonat bei Raumtemperatur während 15 Stunden, sowie Aufarbeiten und Chromatographie des Rückstands wie im Beispiel 2 beschrieben; Rf-Wert im System Methylenchlorid/Methanol (9:1) auf Kieselgel 0,15.

e) N-Propionyl-L-glutaminsäure-5-[$N^2$-(5-n-butyl-2-pyridyl)-hydrazid] durch Umsetzung des nach Beispiel 1 erhaltenen L-Glutaminsäure-5-[$N^2$-(5-n-butyl-2-pyridyl)-hydrazid]-hemihydrat mit Propionsäureanhydrid in Dimethylformamid in Gegenwart von wasserfreiem Natriumcarbonat bei Raumtemperatur während 20 Stunden, Aufarbeiten und Chromatographie des Rückstands über Kieselgel sowie Weiterverarbeiten der Hauptfraktionen wie im Beispiel 2 beschrieben; Rf-Wert im System Methylenchlorid/Methanol/$NH_4OH$ konz. (5:3:1) 0,78.

f) N-Pivaloyl-L-glutaminsäure-5-[$N^2$-(5-n-butyl-2-pyridyl)-hydrazid] durch Umsetzung des nach Beispiel 1 erhaltenen L-Glutaminsäure-5-[$N^2$-(5-n-butyl-2-pyridyl)-hydrazid]-hemihydrat mit Pivalinsäureanhydrid in Dimethylformamid in Gegenwart von wasserfreiem Natriumcarbonat bei einer Temperatur von 40-50° während 20 Stunden, Aufarbeiten und Chromatographie des Rückstands über Kieselgel sowie Weiterverarbeiten der Hauptfraktionen wie im Beispiel 2 beschrieben; Rf-Wert im System Methylenchlorid/Methanol/$NH_4OH$ konz. (5:3:1) 0,80.

g) N-Benzoyl-L-glutaminsäure-5-[$N^2$-(5-n-butyl-2-pyridyl)-hydrazid] durch Umsetzung des nach Beispiel 1 erhaltenen L-Glutaminsäure-5-[$N^2$-(5-n-butyl-2-pyridyl)-hydrazid]-hemihydrat mit Benzoesäureanhydrid in Dimethylformamid in Gegenwart von wasserfreiem Natriumcarbonat bei einer Temperatur von 40 - 50° während 25 Stunden, Aufarbeiten und Chromatographie des Rückstands über Kieselgel sowie Weiterverarbeiten der Hauptfraktionen wie im Beispiel 2 beschrieben; Rf-Wert im

BAD ORIGINAL

System Methylenchlorid/Methanol/$NH_4OH$ konz. (5:3:1) 0,79.

h) N-Acetyl-L-glutaminsäure-5-[$N^2$-(5-methyl-2-pyridyl)-hydrazid] durch Umsetzung von 2-Hydrazino-5-methyl-pyridin mit N-(Benzyloxycarbonyl)-L-glutaminsäure-1-benzylester-5-(N-hydroxysuccinimid)-ester in wasserfreiem Tetrahydrofuran bei 0° während 2 Stunden, Abdampfen des Lösungsmittels und Chromatographie des Rückstands über Kieselgel unter Verwendung von Essigsäureäthylester als Laufmittel und Aufarbeiten der Hauptfraktionen zum (N-Benzyloxycarbonyl)-L-glutaminsäure-5-[$N^2$-(5-methyl-2-pyridyl)-hydrazid]-1-benzylester, dessen anschliessende Entbenzylierung mittels Wasserstoff in Methanol in Gegenwart eines Palladium-auf-Kohle-Katalysators (5 %), Umkristalliseren des nach dem Aufarbeiten erhaltenen L-Glutaminsäure-5-[$N^2$-(5-methyl-2-pyridyl)-hydrazid] aus Methanol, und dessen N-Acetylierung durch Umsetzung mit Essigsäureanhydrid in Dimethylformamid in Gegenwart von wasserfreiem Natriumcarbonat während 15 Stunden bei Raumtemperatur, Abdampfen des Lösungsmittels und Chromatographie des Rückstands über Kieselgel mittels eines Gemisches von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,70.

i) N-Benzoyl-L-glutaminsäure-5-[$N^2$-(5-methyl-2-pyridyl)-hydrazid] durch Umsetzung des nach Beispiel h) erhaltenen L-Glutaminsäure-5-[$N^2$-(5-methyl-2-pyridyl)-hydrazid] mit Benzoesäureanhydrid in Dimethylformamid in Gegenwart von wasserfreiem Natriumcarbonat während 20 Stunden bei Raumtemperatur, Abdampfen des Lösungsmittels, Chromatographie des Rückstands über Kieselgel mittels eines Gemischs von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,73.

i) N-Acetyl-L-glutaminsäure-5-[$N^2$-(5-benzyl-2-pyridyl)-hydrazid] durch Umsetzung von 2-Hydrazino-5-benzyl-pyridin mit N-(Benzyloxycarbonyl)-L-glutaminsäure-1-benzylester-5-(N-hydroxysuccinimid)-ester in wasserfreiem Tetrahydrofuran bei 0° während 2 Stunden, Abdampfen des Lösungsmittels und Chromatographie des Rückstands über Kieselgel unter Verwendung von Essigsäureäthylester als Laufmittel und Aufarbeiten der Hauptfraktionen zum (N-Benzyloxycarbonyl)-L-glutaminsäure-5-[$N^2$-(5-benzyl-2-pyridyl)-hydrazid]-1-benzylester, dessen anschliessende Entbenzylierung mittels Wasserstoff in Methanol in Gegenwart eines Palladium-auf-Kohle-Katalysators (5 %), Umkristallisieren des nach dem Aufarbeiten erhaltenen L-Glutaminsäure-5-[$N^2$-(5-benzyl-2-pyridyl)-hydrazid] aus Methanol, und dessen N-Acetylierung durch Umsetzung mit Essigsäureanhydrid in Dimethylformamid in Gegenwart von wasserfreiem Natriumcarbonat während 15 Stunden bei Raumtemperatur, Abdampfen des Lösungsmittels und Chromatographie des Rückstands über Kieselgel mittels eines Gemischs von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,81.

k) N-Acetyl-L-glutaminsäure-5-[$N^2$-(5-phenyl-2-pyridyl)-hydrazid] durch Umsetzung von 2-Hydrazino-5-phenyl-pyridin mit N-(Benzyloxycarbonyl)-L-glutaminsäure-1-benzylester-5-(N-hydroxysuccinimid)-ester in wasserfreiem Tetrahydrofuran bei 0° während 2 Stunden, Abdampfen des Lösungsmittels und Chromatographie des Rückstand über Kieselgel unter Verwendung von Essigsäureäthylester als Laufmittel und Aufarbeiten der Hauptfraktionen zum (N-Benzyloxycarbonyl)-L-glutaminsäure-5-[$N^2$-(5-phenyl-2-pyridyl)-hydrazid]-1-benzylester, dessen anschliessende Entbenzylierung mittels Wasserstoff in Methanol in Gegenwart eines Palladium-auf-Kohle-Katalysators (5 %), Umkristallisieren des nach dem Aufarbeiten erhaltenen L-Glutaminsäure-5-[$N^2$-(5-phenyl-2-pyridyl)-hydrazid] aus Methanol, und dessen N-Acetylierung durch Umsetzung

mit Essigsäureanhydrid in Dimethylformamid in Gegenwart von wasserfreiem Natriumcarbonat während 15 Stunden bei Raumtemperatur, Abdampfen des Lösungsmittels und Chromatographie des Rückstands über Kieselgel mittels eines Gemischs von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,79.

l) N-Acetyl-L-glutaminsäure-5-[$N^2$-(5-(2-oxo-n-butyl)-2-pyridyl)-hydrazid durch Umsetzung von 2-Hydrazino-5-(2-oxo-n-butyl)-pyridin mit N-(Benzyloxycarbonyl)-L-glutaminsäure-1-benzylester-5-(N-hydroxysuccinimid)-ester in wasserfreiem Tetrahydrofuran bei 0° während 2 Stunden, Abdampfen des Lösungsmittels und Chromatographie des Rückstands über Kieselgel unter Verwendung von Essigsäureäthylester als Laufmittel und Aufarbeiten der Hauptfraktionen zum (N-Benzyloxycarbonyl)-L-glutaminsäure-5-[$N^2$-(5-(2-oxo-n-butyl-2-pyridyl)-hydrazid]-1-benzylester, dessen anschliessende Entbenzylierung mittels Wasserstoff in Methanol in Gegenwart eines Palladium-auf-Kohle-Katalysators (5 %), Umkristallisieren des nach dem Aufarbeiten erhaltenen L-Glutaminsäure-5-[$N^2$-(5-(2-oxo-n-butyl)-2-pyridyl)-hydrazid] aus Methanol/Aethanol-Gemisch und dessen N-Acetylierung durch Umsetzung mit Essigsäureanhydrid in Dimethylformamid in Gegenwart von wasserfreiem Natriumcarbonat während 15 Stunden bei Raumtemperatur, Abdampfen des Lösungsmittels und Chromatographie des Rückstands über Kieselgel mittels eines Gemisches von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,71.

m) N-Acetyl-L-glutaminsäure-5-[$N^2$-(5-acetyl-2-pyridyl)-hydrazid durch Umsetzung von 2-Hydrazino-5-acetyl-pyridin mit N-(Benzyloxycarbonyl)-L-glutaminsäure-1-benzylester-5-(N-hydroxysuccinimid)-ester in wasserfreiem Tetrahydrofuran

BAD ORIGINAL

bei 0° während 2 Stunden, Abdampfen des Lösungsmittels und Chromatographie des Rückstands über Kieselgel unter Verwendung von Essigsäureäthylester als Laufmittel und Aufarbeiten der Hauptfraktionen zum (N-Benzyloxycarbonyl)-L-glutaminsäure-5-[$N^2$-(5-acetyl-2-pyridyl)-hydrazid]-1-benzylester, dessen anschliessende Entbenzylierung mittels Wasserstoff in Methanol in Gegenwart eines Palladium-auf-Kohle-Katalysators (5 %), Umkristallisieren des nach dem Aufarbeiten erhaltenen L-Glutaminsäure-5-[$N^2$-(5-acetyl-2-pyridyl)-hydrazid] aus Methanol, und dessen N-Acetylierung durch Umsetzung mit Essigsäureanhydrid in Dimethylformamid in Gegenwart von wasserfreiem Natriumcarbonat während 15 Stunden bei Raumtemperatur, Abdampfen des Lösungsmittels und Chromatographie des Rückstands über Kieselgel mittels eines Gemisches von Chloroform/Methanol/Ammoniak 5:3:1 und Aufarbeiten der Hauptfraktionen; Rf-Wert im System Methylenchlorid/Methanol/$NH_4OH$ konz. (5:3:1) 0,65.

n) N-Acetyl-L-Glutaminsäure-5-[$N^2$-(5-n-butyl-2-pyridyl)-hydrazid]-1-(pivaloyloxymethyl)-ester durch Umsetzung des gemäss Beispiel 3 als Zwischenprodukt erhaltenen N-Acetyl-L-glutaminsäure-5-[$N^2$-(5-n-2-pyridyl)-hydrazid, und dessen durch Neutralisation mit Natronlauge und Abdampfen zur Trockne erhaltenen Natriumsalzes mit Pivalinsäurejodmethylester in Dimethylformamid bei einer Temperatur von 0-50° und anschliessendem Rühren während 17 Stunden bei Raumtemperatur, Einengen des Reaktionsgemisches im Hochvakuum, Extrahieren mit Essigsäureäthylester und Flash-chromatographie des nach Abdampfen des Lösungsmittels erhaltenen Rückstandes mit Methylenchlorid/Methanol-Gemisch 9:1; Rf-Wert im System Methylenchlorid/Methanol (9:1) auf Kieselgel 0,26.

In für die Herstellung der unter h) – n) bezeichneten Verbindungen analoger Weise können auch folgende Verbindungen über die entsprechenden Zwischenstufen hergestellt werden:

o) N-Acetyl-L-glutaminsäure-5-[$N^2$-(3-n-butyl-2-pyridyl)-hydrazid] aus 2-Hydrazino-3-n-butyl-pyridin, und dem daraus durch Umsetzung mit N-(Benzyloxycarbonyl)-L-glutaminsäure-1-benzylester-5-(N-hydroxysuccinimid)-ester erhältlichen (N-Benzyloxycarbonyl)-L-glutaminsäure-5-[$N^2$-(3-n-butyl-2-pyridyl)-hydrazid], dessen Entbenzylierung zum L-Glutaminsäure-5-[$N^2$-(3-n-butyl-2-pyridyl)-hydrazid und dessen Acetylierung zur oben genannten N-Acetylverbindung; Rf-Wert im System Methylenchlorid/Methanol/$NH_4OH$ konz. (5:3:1) 0,78.

p) N-Acetyl-L-glutaminsäure-5-[$N^2$-(4-n-butyl-2-pyridyl)-hydrazid] aus 2-Hydrazino-4-n-butyl-pyridin, und dem daraus durch Umsetzung mit N-(Benzyloxycarbonyl)-L-glutaminsäure-1-benzylester-5-(N-hydroxysuccinimid)-ester erhältlichen (N-Benzyloxycarbonyl)-L-glutaminsäure-L-Glutaminsäure-5-[$N^2$-(4-n-butyl-2-pyridyl)-hydrazid und dessen Acetylierung zur oben genannten N-Acetylverbindung; Rf-Wert im System Methylenchlorid/Methanol/$NH_4OH$ konz. (5:3:1) 0,77.

q) N-Acetyl-L-glutaminsäure-5-[$N^2$-(6-n-butyl-2-pyridyl)-hydrazid] aus 2-Hydrazino-6-n-butyl-pyridin, und dem daraus durch Umsetzung mit N-(Benzyloxycarbonyl)-L-glutaminsäure-1-benzylester-5-(N-hydroxysuccinimid)-ester erhältlichen (N-Benzyloxycarbonyl)-L-glutaminsäure-5-[$N^2$-(6-n-butyl-2-pyridyl)-hydrazid], dessen Entbenzylierung zum L-Glutaminsäure-5-[$N^2$-(6-n-butyl-2-pyridyl)-hydrazid und dessen Acetylierung zur oben genannten N-Acetylverbindung; Rf-Wert im System Methylenchlorid/Methanol/$NH_4OH$ konz. (5:3:1) 0,77.

r) L-Alanyl-[$N^2$-(5-n-butyl-2-pyridyl)-hydrazid] durch Umsetzung von 2-Hydrazino-5-n-butylpyridin mit L-Alaninmethylester durch 2-stündiges Erhitzen unter Rückfluss in Benzol in einer Stickstoffatmosphäre, Abdampfen des Lösungsmittels, Lösen des Rückstands in Toluol, Waschen mit Wasser, und Extrahieren der organischen Phase mit 2-n. Salzsäure, Waschen der wässrigen-sauren Lösung mit Aether, Versetzen der wässrigen Phase mit konzentrierter Natronlauge bis zur stark alkalischen Reaktion, und Extrahieren des Gemischs mit Aether,

Waschen der Aetherlösung mit Wasser und Versetzen der mittels Natriumsulfat getrockneten Aetherlösung mit einer 5-n. Lösung von Chlorwasserstoff in Aether bis zur stark sauren Reaktion und Abtrennen des erhaltenen L-Alanyl-[$N^2$-(5-n-butyl-2-pyridyl)-hydrazid]-dihydrochlorids in Form weisser Kristalle; Rf-Wert der freien Base im System Methylenchlorid/Methanol/$NH_4OH$ konz. (5:3:1) 0,92.

s) L-Alanyl-[$N^2$-(6-n-butyl-2-pyridyl)-hydrazid] durch Umsetzung von 2-Hydrazino-6-n-butylpyridin mit L-Alaninmethylester und anschliessendes Aufarbeiten wie unter r) beschrieben und Abtrennen des erhaltenen L-Alanyl-[$N^2$-(6-n-butyl-2-pyridyl)-hydrazid]-dihydrochlorid in Form weisser Kristalle; Rf-Wert der freien Base im System Methylenchlorid/Methanol/$NH_4OH$ konz. (5:3:1) 0,90.

t) N-Acetyl-L-alanyl-[$N^2$-(5-n-butyl-2-pyridyl)-hydrazid durch Umsetzen des gemäss q) erhaltenen L-Alanyl-[$N^2$-(5-n-butyl-2-pyridyl)-hydrazid]-dihydrochlorids mit konzentrierter Natronlauge und der so erhaltenen Base mit Essigsäureanhydrid in Dimethylformamid in Gegenwart von wasserfreiem Natriumcarbonat bei Raumtemperatur während 10 Stunden, Abdampfen des Lösungsmittels unter vermindertem Druck, Aufnehmen des Rückstands in Aether, Waschen der Aetherlösung mit Wasser, Versetzen der Aetherlösung mit einer 5-n. Lösung von Chlorwasserstoff bis zur stark sauren Reaktion und Abtrennen des erhaltenen N-Acetyl-L-alanyl-[$N^2$-(5-n-butyl-2-pyridyl)-hydrazid-hydrochlorid in Form weisser Kristalle; Rf-Wert der freien Base im System Methylenchlorid/Methanol/$NH_4OH$ konz. (5:3:1) 0,85.

u) N-Acetyl-L-alanyl-[$N^2$-(6-n-butyl-2-pyridyl)-hydrazid] durch Umsetzen des gemäss s) erhaltenen L-Alanyl-[$N^2$-(6-n-butyl-2-pyridyl)-hydrazid-dihydrochlorids mit konzentrierter Natronlauge, Umsetzen der so erhaltenen Base mit Essigsäureanhydrid analog wie unter s) beschrieben, Aufarbeiten des Reaktionsgemischs und Abtrennen des erhaltenen N-Acetyl-L-alanyl-[$N^2$-(6-n-butyl-2-pyridyl)-hydrazid]-hydrochlorids in Form weisser Kristalle; Rf-Wert der freien Base im System Methylenchlorid/Methanol/$NH_4OH$ konz. (5:3:1) 0,83.

v) Aus der durch Behandeln des gemäss r) erhaltenen L-Alanyl-[$N^2$-(5-n-butyl-2-pyridyl)-hydrazid]-dihydrochlorid mit konzentrierter Natronlauge erhaltenen freien Base kann durch Umsetzung mit den entsprechenden Säureanhydriden analog t) folgende Verbindung in Form ihres Hydrochlorids erhalten werden:

N-Propionyl-L-alanyl-[$N^2$-(5-n-butyl-2-pyridyl)-hydrazid]; Rf-Wert der freien Base im System Methylenchlorid/Methanol/$NH_4OH$ konz. (5:3:1) 0,85.

w) N-Benzoyl-L-alanyl-[$N^2$-(6-methyl-2-pyridyl)-hydrazid] durch Umsetzung von 2-Hydrazino-6-methylpyridin mit L-Alaninmethylester durch 3-stündiges Erhitzen unter Rückfluss in Benzol in einer Stickstoffatmosphäre, Abdampfen des Lösungsmittels, Lösen des Rückstands in Toluol, Waschen mit Wasser, und Extrahieren der organischen Phase mit 2-n. Salzsäure, Waschen der wässrigen-sauren Lösung mit Aether, Versetzen der wässrigen Phase mit konzentrierter Natronlauge bis zur stark alkalischen Reaktion, und Extrahieren des Gemischs mit Aether, Waschen der Aetherlösung mit Wasser und Versetzen der mittels Natriumsulfat getrockneten Aetherlösung mit einer 5-n. Lösung von Chlorwasserstoff in Aether bis zur stark sauren Reaktion und Abtrennen des erhaltenen L-Alanyl-[$N^2$-(6-methyl-2-pyridyl)-hydrazid]-dihydrochlorids in Form weisser Kristalle; Behandeln einer wässrigen Lösung des erhaltenen Dihydrochlorids mit konzentrierter Natriumhydroxidlösung bis zur stark alkalischen Reaktion, Ausschütteln der freien Base mit Aether, Waschen der Aetherlösung mit Wasser, Trocknen über Natriumsulfat und Abdampfen des Lösungsmittels, Umsetzen des aus L-Alanyl-[$N^2$-(6-methyl-2-pyridyl)-hydrazid] bestehenden Rückstands mit Benzoesäureanhydrid in Dimethylformamid in Gegenwart von wasserfreiem Natriumcarbonat während 24 Stunden bei Raumtemperatur und Aufarbeiten des Reaktionsgemischs zum N-Benzoyl-L-alanyl-[$N^2$-(6-methyl-2-pyridyl)-hydrazid]; Rf-Wert im System Methylenchlorid/Methanol/$NH_4OH$ konz. (5:3:1) 0,80.

Beispiel 11: Tabletten enthaltend 20 mg an aktiver Substanz werden in folgender Zusammensetzung in üblicher Weise hergestellt:

Zusammensetzung:

| | |
|---|---|
| Ammoniumsalz des N-Acetyl-L-Glutaminsäure-5-[$N^2$-(5-n-butyl-2-pyridyl)-hydrazid] | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Das Ammoniumsalz des N-Acetyl-L-glutaminsäure-5-[$N^2$-(5-n-butyl-2-pyridyl)-hydrazid] wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 12: Tabletten enthalten 1 mg an aktiver Substanz werden in folgender Zusammensetzung in üblicher Weise hergestellt:

BAD ORIGINAL

Zusammensetzung:

| | |
|---|---|
| L-Glutaminsäure-5-[$N^2$-(5-n-butyl-2-pyridyl)-hydrazid]-hemihydrat | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

L-Glutaminsäure-5-[$N^2$-(5-n-butyl-2-pyridyl)-hydrazid]-hemihydrat wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 13: Kapseln enthaltend 10 mg an aktiver Substanz werden wie folgt auf übliche Weise hergestellt:

Zusammensetzung:

| | |
|---|---|
| $N^2$-(5-n-Butyl-2-pyridyl)-3-sulfamoyl-4-chlor-benzoesäure-hydrazid-acetat | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sie mit 0,5 mm Maschenweite getrieben und dieses in Portionen von jeweils 11 mg in Hartgelatinekapseln geeigneter Grösse abgefüllt.

Beispiel 14: Eine sterile Lösung von 5,0 g Ammoniumsalz des N-Acetyl-L-glutaminsäure-5-[$N^2$-(5-n-butyl-2-pyridyl)-hydrazid in 5000 ml destilliertem Wasser wird in Ampullen zu 5 ml abgefüllt, die in 5 ml Lösung 5 mg Wirkstoff enthalten.

Beispiel 15: 3,62 g $N^2$-(5-n-Butyl-2-pyridyl)-3-sulfamoyl-4-chlorbenzoesäurehydrazidacetat werden mit destilliertem Wasser auf ein Volumen von 18100 ml gelöst. Die sterilisierte Lösung wird in Ampullen à 5,0 ml abgefüllt, in denen 1 mg Wirkstoff enthalten ist.

Beispiel 16: Anstelle der in den Beispielen 11 bis 15 als aktive Substanz verwendeten Verbindungen können auch folgende Verbindungen der Formel I oder deren pharmazeutisch verwendbaren, nicht-toxischen Säureadditionssalze als Wirkstoffe in Tabletten, Dragées, Kapseln etc. verwendet werden:
$N^2$-(5-n-Butyl-2-pyridyl)-picolinsäurehydrazid, N-Aethoxycarbonyl-$N^2$-(5-n-butyl-2-pyridyl)-hydrazin, $N^2$-(5-n-Butyl-2-pyridyl)-N-essigsäurehydrazid oder $N^2$-(5-n-Butyl-2-pyridyl)-N-fusarinsäurehydrazid, N-Acetyl-L-glutaminsäure-5-[$N^2$-(5-n-butyl-2-pyridyl)-hydrazid]-1-methylester, oder die im Beispiel 10 beschriebenen Verbindungen der Formel I oder deren Salze, insbesondere pharmazeutisch verwendbaren, nicht-toxischen Säureadditionssalze.

Patentansprüche:

1. Pharmazeutische Präparate enthaltend Verbindungen der Formel

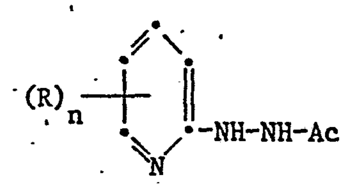

(I) ,

worin R einen gegebenenfalls substituierten aliphatischen oder aromatischen Kohlenwasserstoffrest und Ac einen Acylrest bedeutet, und n eine ganze Zahl von 1-4 darstellt, wobei, falls n einen Wert von 2-4 bedeutet, die Reste R gleich oder verschieden und in jeder der möglichen Stellungen im Pyridinring gebunden sein können, als Racematgemische, Racemate, optische Antipoden oder deren Salze, insbesondere deren pharmazeutisch verwendbaren nicht-toxischen Säureadditionssalze.

2. Pharmazeutische Präparate enthaltend Verbindungen der Formel I gemäss Anspruch 1, worin n eine ganze Zahl von 1-4 darstellt und R gegebenenfalls substituiertes Niederalkyl, Niederalkenyl oder Niederalkinyl, oder einen mono- oder bicyclischen, carbocyclischen oder heterocyclischen aromatischen Kohlenwasserstoffrest bedeutet, Ac einen Rest der Formel $-C(=O)-R_1$ darstellt, worin $R_1$ einen gegebenenfalls substituierten aliphatischen, cycloaliphatischen, aromatischen oder araliphatischen Kohlenwasserstoffrest, oder Ac den Rest eines Monoesters oder Monoamids der Kohlensäure bedeutet, als Racematgemische, Racemate, optische Antipoden oder deren Salze, insbesondere deren pharmazeutisch verwendbaren, nicht-toxischen Säureadditionssalze.

3. Pharmazeutische Präparate enthaltend Verbindungen der Formel I gemäss Anspruch 1, worin n eine ganze Zahl von 1-4 darstellt und R

Niederalkyl, gegebenenfalls veräthertes oder verestertes Hydroxyniederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl, wie Aminoniederalkyl, N-Niederalkylaminoniederalkyl oder N,N-Diniederalkylaminoniederalkyl, Niederalkanoylaminoniederalkyl oder Niederalkoxycarbonylaminoniederalkyl, durch gegebenenfalls verestertes oder amidiertes Carboxy, wie Carboxy, Aethoxycarbonyl oder gegebenenfalls substituiertes Carbamoyl, wie Carbamoyl, substituiertes Niederalkyl, wie Carboxyniederalkyl, Niederalkoxycarbonylniederalkyl, Carbamoylniederalkyl, Oxo-niederalkyl, ferner Cyanniederalkyl, gegebenenfalls substituiertes Arylniederalkyl, worin Aryl ein monocyclischer, aromatischer Kohlenwasserstoffrest, z.B. Phenyl, oder ein bicyclischer aromatischer Kohlenwasserstoffrest, wie 1- oder 2-Naphthyl, oder teilweise gesättigtes Naphthyl, wie 1,2,3,4-Tetrahydro-5-naphthyl ist, wobei Substituenten Niederalkyl, Hydroxy und/oder Halogen, Hydroxyniederalkyl, Niederalkoxyniederalkyl, Halogenniederalkyl, Niederalkoxy, Niederalkenyloxy, oder gegebenenfalls niederalkyliertes oder acyliertes Aminoniederalkyl, z.B. Mono- oder Diniederalkylaminoniederalkyl oder Niederalkanoylaminoniederalkyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls acyliertes Amino, z.B. Amino, Niederalkanoylamino, Niederalkoxycarbonylamino, Niederalkylamino oder Diniederalkylamino sein und solche Substituenten gleich oder verschieden und [illegible] vorhanden sein können, bedeutet, oder R und das in der Gruppe [illegible] jeweils für Aryl, wie Phenyl, 1- oder 2-Naphthyl oder teilweise gesättigtes Naphthyl, wie 1,2,3,4-Tetrahydro-5-naphthyl oder einen über ein Ringkohlenstoffatom mit dem Pyridylrest verbundenen, ein Sauerstoff-, Schwefel- oder Stickstoffatom und gegebenenfalls 1-3 zusätzliche Stickstoffatome als Ringglieder enthaltenden Rest,

BAD ORIGINAL

vorzugsweise mit 5 oder 6 Ringgliedern steht, der mindestens partiell hydriert und in diesem Fall durch Oxo substituiert sein kann, wobei in einem bicyclischen heterocyclischen Rest der zweite Ring ein ankondensierter Benzoring sein kann, und insbesondere Pyridyl, z.B. 2-, 3- oder 4-Pyridyl, oder Dihydro-oxo-pyridyl z.B. 1,6-Dihydro-6-oxo-2-pyridinyl, Pyridazinyl, z.B. 3-Pyridazinyl, Pyrazinyl, z.B. 2-Pyrazinyl, Pyrimidinyl, z.B. 2-, 4- oder 5-Pyrimidinyl, oder Dihydro-oxo-pyrimidinyl z.B. 3,4-Dihydro-4-oxo-2-pyrimidinyl, Furyl z.B. 2- oder 3-Furyl, Pyrryl z.B. 2- oder 3-Pyrryl, Thienyl z.B. 2- oder 3-Thienyl, Oxazolyl z.B. 2- oder 4-Oxazolyl, Thiazolyl, z.B. 2-, 4- oder 5-Thiazolyl, Thiadiazolyl z.B. 1,2,4-Thiadiazol-3- oder -5-yl oder 1,2,5-Thiadiazol-3-yl, Imidazolyl z.B. Imidazol-2- oder -4-yl, Pyrazolyl z.B. 3- oder 4-Pyrazolyl, Triazolyl, z.B. 1,2,3-Triazol-4-yl oder 1,2,4-Triazol-3-yl, Tetrazolyl wie Tetrazol-5-yl, ferner Indolyl z.B. Indol-4-yl, Chinolinyl, z.B. 2-Chinolinyl, Isochinolinyl, z.B. 1-Isochinolinyl, Benzimidazolyl, z.B. 2-Benzimidazolyl, Benzofuranyl, z.B. 4- oder 5-Benzofuranyl, Benzothienyl, z.B. 4- oder 5-Benzothienyl, oder Naphthyridinyl z.B. 1,8-Naphthyridin-2-yl bedeutet, wobei solche Arylreste in erster Linie an einem Ringkohlenstoffatom, aber auch einem sekundären Ringstickstoffatom stehen, und ein- oder mehrfach, vorzugsweise höchstens vierfach, durch gegebenenfalls substituiertes Niederalkyl, z.B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl wie Aminoniederalkyl, N-Niederalkylaminoniederalkyl, N,N-Diniederalkylaminoniederalkyl, Niederalkanoylaminoniederalkyl oder Niederalkoxycarbonylaminoniederalkyl, oder Niederalkenyl oder Niederalkinyl, gegebenenfalls veräthertes oder verestertes Hydroxy oder Mercapto, wie Hydroxy, Niederalkoxy, Phenylniederalkoxy, Halogen, Mercapto, Niederalkylthio, Phenylniederalkylthio, durch gegebenenfalls veräthertes oder verestertes Hydroxy oder Mercapto oder durch Acyl substituiertes Niederalkoxy, z.B. Niederalkoxy,

Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkinyloxy, Niederalkylthio, Acyl, z.B. Niederalkanoyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Niederalkanoylamino, Niederalkoxycarbonylamino, Niederalkylsulfonyl, Sulfamoyl, gegebenenfalls substituiertes Ureido, ferner Amino, N-Niederalkylamino oder N,N-Diniederalkylamino, substituiert sein können, das in einer Gruppe Ac enthaltene $R_1$ ausserdem gegebenenfalls durch gegebenenfalls veräthertes oder verestertes Hydroxy, wie Hydroxy, Niederalkoxy und/oder Halogen, gegebenenfalls substituiertes wie niederalkyliertes oder acyliertes Amino, wie Amino, N-Niederalkylamino oder N,N-Diniederalkylamino, Niederalkanoylamino und/oder gegebenenfalls funktionell abgewandeltes Carboxy, wie Carboxy, verestertes Carboxy, z.B. gegebenenfalls substituiertes Niederalkoxycarbonyl, gegebenenfalls substituiertes Carbamoyl, oder Cyan, wobei substituierte Reste einen oder mehrere Substituenten die gleich oder verschieden und 1-3-fach vorhanden sein können, in irgendwelchen der zur Substitution geeigneten Stellungen aufweisen können, substituiertes Niederalkyl, Niederalkenyl, Niederalkinyl, Phenylniederalkyl, Phenylniederalkenyl, Phenylniederalkinyl, mono- oder polycyclisches Cycloalkyl, etwa Cycloalkyl, bedeutet, oder Ac ausserdem unsubstituiertes oder substituiertes, z.B. freies oder veräthertes Hydroxy, wie Niederalkoxy, oder unsubstituiertes oder substituiertes Amino, wie Amino, Niederalkylamino, und in erster Linie disubstituiertes Amino, wie Diniederalkylamino, N-Niederalkyl-N-phenylniederalkylamino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder

Halogen substituiertes Phenyl enthaltendes Niederalkoxycarbonyl, ferner N-unsubstituiertes oder N-mono- oder N,N-disubstituiertes Carbamoyl, wie Niederalkylcarbamoyl, Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylencarbamoyl darstellt, als Racematgemische, Racemate, optische Antipoden oder deren Salze, insbesondere deren pharmazeutisch verwendbare, nicht-toxischen Säureadditionssalze.

4. Pharmazeutische Präparate enthaltend Verbindungen der Formel I, gemäss Anspruch 1, worin n für 1 oder 2 steht und R Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. Acetylaminomethyl bedeutet, und $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. 2-Acetylaminoäthyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Niederalkanoylamino, z.B. Acetylamino, oder Sulfamoyl substituiertes Phenyl oder Pyridyl, ferner gegebenenfalls durch Niederalkoxy, z.B. Methoxy, Amino, Niederalkanoylamino, z.B. Acetylamino, Carboxy, gegebenenfalls substituiertes Niederalkoxycarbonyl, z.B. Aethoxycarbonyl, oder 2- oder vorzugsweise 1-(Niederalkanoyloxy)-niederalkoxycarbonyl oder Carbamoyl substituiertes Niederalkyl, z.B. Aethyl oder n-Propyl bedeutet, oder Ac ausserdem unsubstituiertes oder durch Niederalkoxy, wie Methoxy, oder Amino substituiertes Niederalkoxycarbonyl, wie Aethoxycarbonyl, oder Carbamoyl darstellt, als Racematgemische, Racemate, optische Antipoden oder deren Salze, insbesondere deren pharmazeutisch verwendbare, nicht-toxischen Säureadditionssalze.

5. Pharmazeutische Präparate enthaltend Verbindungen der Formel I, gemäss Anspruch 1, worin n für 1 steht, und R Niederalkyl, z.B. Methyl, Niederalkoxyniederalkyl, z.B. 2-Methoxyäthyl, Halogenniederalkyl, z.B. Trifluormethyl, $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Sulfamoyl substituiertes Phenyl oder Pyridyl, ferner gegebenenfalls durch Niederalkoxy, z.B. Methoxy, Amino, Niederalkanoylamino, z.B. Acetylamino und Carboxy oder gegebenenfalls substituiertes Niederalkoxycarbonyl,z.B.Aethoxycarbonyl, ferner 2- oder vorzugsweise 1-(Niederalkylcarboxyloxy)-niederalkoxycarbonyl, oder Carbamoyl substituiertes Niederalkyl,z.B.Aethyl oder n-Propyl bedeutet,oder Ac ausserdem Niederalkoxycarbonyl,wie Aethoxycarbonyl,oder Carbamoyl darstellt, als Racematgemische, Racemate, optische Antipoden oder deren Salze, insbesondere durch pharmazeutisch verwendbare, nicht-toxischen Säureadditionssalze.

6. Pharmazeutische Präparate enthaltend Verbindungen der Formel I, gemäss Anspruch 1, worin n für 1 steht, und R Niederalkyl, z.B. Methyl, Niederalkoxyniederalkyl, z.B. 2-Methoxyäthyl, $R_1$ in einer Gruppe Ac gegebenenfalls durch Niederalkyl, z.B. Methyl, Halogenniederalkyl, z.B. Trifluormethyl, Hydroxy, Niederalkoxy, z.B. Methoxy, Halogen, Carbamoyl, Cyano oder und/oder Sulfamoyl substituiertes Phenyl, ferner gegebenenfalls durch Amino Niederalkanoylamino, z.B. Acetylamino, und Carboxy oder gegebenenfalls substituiertes Niederalkoxycarbonyl, wie Niederalkoxycarbonyl, z.B. Aethoxycarbonyl oder 2- oder vorzugsweise 1-(Niederalkylcarboxyloxyniederalkoxycarbonyl substituiertes Niederalkyl, z.B.Aethyl,n-Propyl oder n-Butyl bedeutet, oder Ac ausserdem Niederalkoxycarbonyl, wie Aethoxycarbonyl,

darstellt, als Racematgemische, Racemate, optische Antipoden oder deren Salze, insbesondere deren pharmazeutisch verwendbare, nichttoxischen Säureadditionssalzen, oder sofern saure Gruppen vorhanden sind, als Salze mit pharmazeutisch verwendbaren, nicht toxischen Basen.

7. Neue Verbindungen der Formel

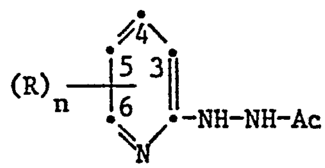

(I) ,

worin R einen gegebenenfalls substituierten aliphatischen oder aromatischen Kohlenwasserstoffrest und Ac einen Acylrest bedeutet, und n eine ganze Zahl von 1-4 darstellt, wobei, falls n einen Wert von 2-4 bedeutet, die Reste R gleich oder verschieden und in jeder der möglichen Stellungen des Pyridinrings gebunden sein können, mit der Massgabe, dass R als in 3-Stellung gebundenes Niederalkyl 2-7 Kohlenstoffatome hat, und n den Wert 1 bedeutet, wenn Ac die Acetylgruppe bedeutet, und mit der weiteren Massgabe, dass R als in 3-, 4-, 5- oder 6-Stellung gebundenes Niederalkyl 2-7 Kohlenstoffatome hat, wenn Ac die Benzoylgruppe bedeutet, als Racematgemische, Racemate oder optische Antipoden.

8. Neue Verbindungen der Formel I gemäss Anspruch 7, worin n eine ganze Zahl von 1-4 darstellt und R gegebenenfalls substituiertes Niederalkyl, Niederalkenyl oder Niederalkinyl, oder einen mono- oder bicyclischen, carbocyclischen oder heterocyclischen aromatischen Kohlenwasserstoffrest bedeutet, Ac einen Rest der Formel $-C(=O)-R_1$ darstellt, worin $R_1$ einen gegebenenfalls substituierten aliphatischen, cycloaliphatischen, aromatischen oder araliphatischen Kohlenwasserstoffrest, oder Ac den Rest eines Monoesters oder Monoamids der Kohlensäure bedeutet, mit der Massgabe, dass R als in 3-Stellung gebundenes Niederalkyl 2-7 Kohlenstoffatome hat, wenn Ac die Acetylgruppe bedeutet, und mit der weiteren Massgabe, dass R als in 3-, 4-, 5- oder 6-Stellung gebundenes Niederalkyl 2-7 Kohlenstoffatome hat,

wenn Ac die Benzoylgruppe bedeutet, als Racematgemische, Racemate oder optische Antipoden.

9. Neue Verbindungen der Formel I gemäss Anspruch 7, worin n eine ganze Zahl von 1-4 darstellt und R Niederalkyl, gegebenenfalls veräthertes oder verestertes Hydroxyniederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl, wie Aminoniederalkyl, N-Niederalkylaminoniederalkyl oder N,N-Diniederalkylaminoniederalkyl, Niederalkanoylaminoniederalkyl oder Niederalkoxycarbonylaminoniederalkyl, durch gegebenenfalls verestertes oder amidiertes Carboxy, wie Carboxy, Aethoxycarbonyl oder gegebenenfalls substituiertes Carbamoyl, wie Carbamoyl, substituiertes Niederalkyl, wie Carboxyniederalkyl, Niederalkoxycarbonylniederalkyl, Carbamoylniederalkyl, Oxo-niederalkyl, ferner Cyanniederalkyl, gegebenenfalls substituiertes Arylniederalkyl, worin Aryl ein monocyclischer, aromatischer Kohlenwasserstoffrest, z.B. Phenyl, oder ein bicyclischer aromatischer Kohlenwasserstoffrest, wie 1- oder 2-Naphthyl, oder teilweise gesättigtes Naphthyl, wie 1,2,3,4-Tetrahydro-5-naphthyl ist, wobei Substituenten Niederalkyl, Hydroxy und/oder Halogen, Hydroxyniederalkyl, Niederalkoxyniederalkyl, Halogenniederalkyl, Niederalkoxy, Niederalkenyloxy, oder gegebenenfalls niederalkyliertes oder acyliertes Aminoniederalkyl, z.B. Mono- oder Diniederalkylaminoniederalkyl oder Niederalkanoylaminoniederalkyl, gegebenenfalls verestertes Carboxy, wie Carboxy oder Niederalkoxycarbonyl amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls acyliertes Amino, z.B. Amino, Niederalkylamino, Niederalkoxycarbonylamino, Niederalkylamino oder Diniederalkylamino sein und solche Substituenten gleich oder verschieden [illegible] 1-3-fach vorhanden sein können, bedeutet, oder R und $R_1$ in der Gruppe Ac jeweils für Aryl, wie Phenyl, 1- oder 2-Naphthyl oder teilweise gesättigtes Naphthyl, wie 1,2,3,4-Tetrahydro-5-naphthyl

oder einen über ein Ringkohlenstoffatom mit dem Pyridylrest verbundenen, ein Sauerstoff-, Schwefel- oder Stickstoffatom und gegebenenfalls 1-3 zusätzliche Stickstoffatome als Ringglieder enthaltenden Rest, vorzugsweise mit 5 oder 6 Ringgliedern steht, der mindestens partiell hydriert und in diesem Fall durch Oxo substituiert sein kann, wobei in einem bicyclischen heterocyclischen Rest der zweite Ring ein ankondensierter Benzoring sein kann, und insbesondere Pyridyl, z.B. 2-, 3- oder 4-Pyridyl, oder Dihydro-oxo-pyridyl z.B. 1,6-Dihydro-6-oxo-2-pyridinyl, Pyridazinyl, z.B. 3-Pyridazinyl, Pyrazinyl, z.B. 2-Pyrazinyl, Pyrimidinyl, z.B. 2-, 4- oder 5-Pyrimidinyl, oder Dihydro-oxo-pyrimidinyl z.B. 3,4-Dihydro-4-oxo-2-pyrimidinyl, Furyl z.B. 2- oder 3-Furyl, Pyrryl z.B. 2- oder 3-Pyrryl, Thienyl z.B. 2- oder 3-Thienyl, Oxazolyl z.B. 2- oder 4-Oxazolyl, Thiazolyl, z.B. 2-, 4- oder 5-Thiazolyl, Thiadiazolyl z.B. 1,2,4-Thiadiazol-3- oder -5-yl oder 1,2,5-Thiadiazol-3-yl, Imidazolyl z.B. Imidazol-2- oder -4-yl, Pyrazolyl z.B. 3- oder 4-Pyrazolyl, Triazolyl, z.B. 1,2,3-Triazol-4-yl oder 1,2,4-Triazol-3-yl, Tetrazolyl wie Tetrazol-5-yl, ferner Indolyl z.B. Inol-4-yl, Chinolinyl, z.B. 2-Chinolinyl, Isochinolinyl, z.B. 1-Isochinolinyl, Benzimidazolyl, z.B. 2-Benzimidazolyl, Benzofuranyl, z.B. 4- oder 5-Benzofuranyl, Benzothienyl, z.B. 4- oder 5-Benzothienyl, oder Naphthyridinyl z.B. 1,8-Naphthyridin-2-yl bedeutet, wobei solche Arylreste in erster Linie an einem Ringkohlenstoffatom, aber auch einem sekundären Ringstickstoffatom stehen und ein- oder mehrfach, vorzugsweise höchstens vierfach, durch gegebenenfalls substituiertes Niederalkyl, z.B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie niederalkyliertes oder acyliertes Aminoniederalkyl wie Aminoniederalkyl, N-Niederalkylaminoniederalkyl, N,N-Diniederalkylaminoniederalkyl, Niederalkanoylaminoniederalkyl oder Niederalkoxycarbonylaminoniederalkyl, oder Niederalkenyl oder Niederalkinyl, gegebenenfalls veräthertes oder verestertes

Hydroxy oder Mercapto, wie Hydroxy, Niederalkoxy, Phenylniederalkoxy, Halogen, Mercapto, Niederalkylthio, Phenylniederalkylthio, durch gegebenenfalls veräthertes oder verestertes Hydroxy oder Mercapto, oder durch Acyl substituiertes Niederalkoxy, z.B. Niederalkoxy, Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkinyloxy, Niederalkylthio, Acyl, z.B. Niederalkanoyl, gegebenenfalls verestertes Carboxy, wie Carboxy oder Niederalkoxycarbonyl, amidiertes Carboxy, z.B. gegebenenfalls substituiertes Carbamoyl, wie Carbamoyl, N-Niederalkylcarbamoyl oder N,N-Diniederalkylcarbamoyl, Cyan, Nitro oder gegebenenfalls substituiertes, wie acyliertes Amino, z.B. Niederalkanoylamino, Niederalkoxycarbonylamino, Niederalkylsulfonyl, Sulfamoyl, gegebenenfalls substituiertes Ureido, ferner Amino, N-Niederalkylamino der N,N-Diniederalkylamino, substituiert sein können, das in einer Gruppe Ac enthaltene $R_1$ ausserdem gegebenenfalls durch gegebenenfalls veräthertes oder verestertes Hydroxy, wie Hydroxy, Niederalkoxy und/oder Halogen, gegebenenfalls substituiertes wie niederalkyliertes oder acyliertes Amino, wie Amino, N-Niederalkylamino oder N,N-Diniederalkylamino, Niederalkanoylamino und/oder gegebenenfalls funktionell abgewandeltes Carboxy, wie Carboxy, verestertes Carboxy, z.B. gegebenenfalls substituiertes Niederalkoxycarbonyl, gegebenenfalls substituiertes Carbamoyl, oder Cyan, wobei substituierte Reste einen oder mehrere Substituenten die gleich oder verschieden und 1-3-fach vorhanden sein können, in irgendwelchen der zur Substitution geeigneten Stellungen aufweisen können, substituiertes Niederalkyl, Niederalkenyl, Niederalkinyl, Phenylniederalkyl, Phenylniederalkenyl, Phenylniederalkinyl, mono- oder polycyclisches Cycloalkyl, etwa Cycloalkyl, bedeutet, oder Ac ausserdem unsubstituiertes oder substituiertes, z.B. freies oder veräthertes Hydroxy, wie Niederalkoxy, oder unsubstituiertes oder substituiertes Amino, wie Amino, Niederalkylamino, und in erster Linie disubstituiertes Amino, wie Diniederalkylamino, N-Niederalkyl-N-phenylniederalkylamino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder Halogen substituiertes Phenyl enthaltendes Niederalkoxycarbonyl, ferner N-unsubstituiertes oder N-mono- oder N,N-disubstituiertes Carbamoyl, wie Niederalkylcarbamoyl, Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylencarbamoyl darstellt, mit der Massgabe, dass R als in 3-Stellung gebundenes Niederalkyl 2-7 Kohlenstoffatome hat, und z.B. Aethyl, n-Propyl oder n-Butyl darstellt, wenn Ac die Acetylgruppe ist und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl bedeutet, wenn Ac die Benzoylgruppe ist, als Racematgemische, Racemate oder optische Antipoden.

10. Neue Verbindungen der Formel I gemäss Anspruch 7, worin n für 1 oder 2 steht und R Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. Acetylaminomethyl, bedeutet, und $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Niederalkanoylaminoniederalkyl, z.B. 2-Acetylaminoäthyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Niederalkanoylamino, z.B. Acetylamino, oder Sulfamoyl substituiertes Phenyl oder Pyridyl, ferner gegebenenfalls durch Niederalkoxy, z.B. Methoxy, Amino, Niederalkanoylamino, z.B. Acetylamino, Carboxy, gegebenenfalls substituiertes Niederalkoxycarbonyl, z.B. Aethoxycarbonyl oder 2- oder 1-(Niederalkylcarbonyloxy)-niederalkoxycarbonyl, oder Carbamoyl substituiertes

Niederalkyl, z.B. Aethyl oder n-Propyl bedeutet, oder Ac ausserdem unsubstituiertes oder durch Niederalkoxy, wie Methoxy, oder Amino substituiertes Niederalkoxycarbonyl, wie Aethoxycarbonyl oder Carbamoyl darstellt, mit der Massgabe, dass R als in 3-Stellung gebundenes Niederalkyl 2-7 Kohlenstoffatome hat, und z.B. Aethyl, n-Propyl oder n-Butyl bedeutet, wenn Ac die Acetylgruppe ist, und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl darstellt, falls Ac die Benzyolgruppe ist, als Racematgemische, Racemate oder optische Antipoden.

11. Neue Verbindungen der Formel I gemäss Anspruch 7, worin n für 1 steht, und R Niederalkyl, z.B. Methyl, Niederalkoxyniederalkyl, z.B. 2-Methoxyäthyl, Halogenniederalkyl, z.B. Trifluormethyl, $R_1$ in der Gruppe Ac gegebenenfalls durch Niederalkyl, z.B. Methyl, Niederalkoxyniederalkyl, z.B. 2-Methoxyäthyl, Halogenniederalkyl, z.B. Trifluormethyl, Hydroxy, Niederalkoxy, z.B. Methoxy, Niederalkoxyniederalkoxy, z.B. 2-Aethoxyäthoxy, Halogen, Niederalkoxycarbonyl, z.B. Aethoxycarbonyl, Carbamoyl, Cyano, Amino oder Sulfamoyl substituiertes Phenyl oder Pyridyl, ferner gegebenenfalls durch Niederalkoxy, Z.B. Methoxy, Amino, Niederalkanoylamino, z.B. Acetylamino, und Carboxy oder gegebenenfalls substituiertes Niederalkoxycarbonyl, z.B. Aethoxycarbonyl oder 2-, vorzugsweise 1-(Niederalkylcarbonyloxy)-niederalkoxycarbonyl, oder Carbamoyl substituiertes Niederalkyl, z.B. Aethyl oder n-Propyl bedeutet, oder Ac ausserdem Niederalkoxycarbonyl, wie Aethoxycarbonyl oder Carbamoyl darstellt, mit der Massgabe, dass R als in 3-Stellung gebundenes Niederalkyl 2-7 Kohlenstoffatome hat, und z.B. Aethyl, n-Propyl oder n-Butyl ist, falls Ac die Acetylgruppe bedeutet, und mit der weiteren Massgabe, dass R als in 3-, 4-, 5- oder 6-Stellung gebundenes Niederalkyl 2-7 Kohlenstoffatome hat, und z.B. vorgenanntes Aethyl, n-Propyl oder n-Butyl bedeutet, falls Ac die Benzoylgruppe ist, als Racematgemische, Racemate oder optische Antipoden.

12. Neue Verbindungen der Formel I gemäss Anspruch 7, worin n für 1 steht, und R Niederalkyl, z.B. Methyl, Niederalkoxyniederalkyl, z.B.

BAD ORIGINAL

2-Methoxyäthyl, $R_1$ in einer Gruppe Ac gegebenenfalls durch Niederalkyl, z.B. Methyl, Halogenniederalkyl, z.B. Trifluormethyl, Hydroxy, Niederalkoxy, z.B. Methoxy, Halogen, Carbamoyl, Cyano und/oder Sulfamoyl substituiertes Phenyl, ferner gegebenenfalls durch Amino, Niederalkanoylamino, z.B. Acetylamino, und Carboxy oder gegebenenfalls substituiertes Niederalkoxycarbonyl, wie Niederalkoxycarbonyl, z.B. Aethoxycarbonyl oder 2-, vorzugsweise 1-(Niederalkylcarbonyloxy)-niederalkoxycarbonyl substituiertes Niederalkyl, z.B. Aethyl, n-Propyl oder n-Butyl bedeutet, oder Ac ausserdem Niederalkoxycarbonyl, wie Aethoxycarbonyl, darstellt, mit der Massgabe, dass R als in 3-Stellung gebundenes Niederalkyl 2-7 Kohlenstoffatome hat und z.B. Aethyl, n-Propyl oder n-Butyl bedeutet, falls Ac die Acetylgruppe ist, und mit der weiteren Massgabe, dass R als in 3-, 4-, 5- oder 6-Stellung gebundenes Niederalkyl 2-7 Kohlenstoffatome hat und z.B. vorgenanntes Aethyl, n-Propyl oder n-Butyl bedeutet, falls Ac die Benzoylgruppe ist, als Racematgemische, Racemate oder optische Antipoden.

13. Neue Verbindungen der Formel I, gemäss Anspruch 7, worin n für 1 steht und R Niederalkyl, z.B. n-Butyl, oder Niederalkoxyniederalkyl, z.B. 3-Methoxy-n-propyl, ist und $R_1$ in der Gruppe Ac gemäss Anspruch 8 durch Amino oder insbesondere Niederalkanoylamino, z.B. Acetylamino, und durch Carboxy, Niederalkoxycarbonyl, z.B. Aethoxycarbonyl, 2- oder vorzugsweise 1-(Niederalkylcarbonyloxy)-nieder alkoxycarbonyl, z.B. Pivalylcarbonyloxy)-methoxycarbonyl, oder Carbamoyl, vorzugsweise in der ω-Stellung, substituiertes Niederalkyl, z.B. Aethyl oder n-Propyl, bedeutet, als Racematgemische, Racemate oder optische Antipoden, oder Salze davon.

14. Neue Verbindungen der Formel I gemäss Anspruch 7, worin n für 1 steht und R in 5-Stellung gebundenes Niederalkyl, z.B. n-Butyl, oder Niederalkoxyniederalkyl, z.B. 3-Methoxy-n-propyl ist, und $R_1$ in der Gruppe Ac gemäss Anspruch 8 in der ω-Stellung durch Amino oder in erster Linie Niederalkanoylamino, z.B. Acetylamino, und durch Carboxy, Niederalkoxycarbonyl, z.B. Aethoxycarbonyl, oder 1-(Niederalkyl-

carbonyloxy)-niederalkoxycarbonyl, z.B. Pivalylcarbonyloxy)-methoxycarbonyl, substituiertes n-Propyl ist, wobei eine Verbindung mit einer solchermassen definierten Gruppe $R_1$ in optisch aktiver Form, insbesondere in der L-Form vorliegt, oder Salze davon.

15. L-Glutaminsäure-5-[$N^2$-(5-n-butyl-2-pyridyl)-hydrazid]-hemihydrat.

16. N-Acetyl-L-glutaminsäure-5-[$N^2$-(5-n-butyl-2-pyridyl)-hydrazid].

17. $N^2$-(5-n-Butyl-2-pyridyl)-3-sulfamoyl-4-chlor-benzoesäurehydrazid.

18. N-Acetyl-L-glutaminsäure-5-[$N^2$-(5-n-butyl-2-pyridyl)-hydrazid]-1-methylester.

19. $N^2$-(5-n-Butyl-2-pyridyl)-picolinsäurehydrazid.

20. N-Aethoxycarbonyl-$N^2$-(5-n-butyl-2-pyridyl)-hydrazin.

21. $N^2$-(5-n-Butyl-2-pyridyl)-N-essigsäurehydrazid.

22. $N^2$-(5-n-Butyl-2-pyridyl)-N-fusarinsäurehydrazid.

23. Die im Beispiel 10 genannten Verbindungen der Formel I.

24. Salze von Verbindungen der Ansprüche 7 bis 23.

25. Pharmazeutisch verwendbare, nicht-toxische Säureadditionssalze von Verbindungen der Ansprüche 7 bis 23.

26. Salze von saure Gruppen enthaltenden Verbindungen der Ansprüche 7 bis 16 mit pharmazeutisch verwendbaren, nicht-toxischen Basen.

27. Pharmazeutische Präparate enthaltend neue Verbindungen der Ansprüche 7 bis 23 und 25 bis 26.

28. Die Gegenstände der Ansprüche 1 bis 23 und 25 bis 26 zur Anwendung in einem Verfahren zur Behandlung des menschlichen oder tierischen Körpers.

29. Die Gegenstände der Ansprüche 1 bis 23 und 25 bis 26 als pharmakologisch aktive, insbesondere im renalen und cardiovaskularen Bereich wirksame pharmazeutische Präparate oder neue Verbindungen.

30. Die Gegenstände der Ansprüche 1 bis 23 und 25 bis 26 als Antihypertensiva.

31. Verbindungen der Formel I, worin Ac den Pyridylcarbonylrest bedeutet, als antileukämisch wirksame Stoffe.

32. Verwendung von Verbindungen der Ansprüche 7 bis 23 und 25 bis 26 zur Herstellung von pharmazeutischen Präparaten.

33. Verfahren zur Herstellung von pharmazeutischen Präparaten, dadurch gekennzeichnet, dass man Verbindungen der Ansprüche 1 bis 23, 25 und 26, gegebenenfalls unter Beimischung von Hilfsstoffen, zu pharmazeutischen Präparaten verarbeitet.

34. Verfahren zur Herstellung von neuen Verbindungen der im Anspruch 7 definierten Formel I, dadurch gekennzeichnet, dass man

a) eine Verbindung der Formel

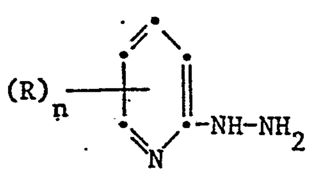

(II)

mit einer Verbindung der Formel

$HOOC-R_1$ (III) ,

oder einem reaktionsfähigen Derivat einer solchen Carbonsäure, oder mit einem reaktionsfähigen Derivat eines Monoesters oder Monoamids der Kohlensäure (IV) umsetzt, oder

b) in einer Verbindung der Formel

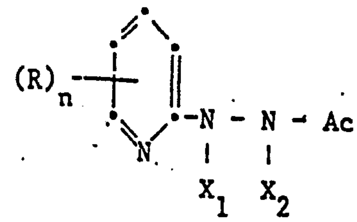

(IV) ,

worin $X_1$ und $X_2$ jeweils Wasserstoff oder einen durch Wasserstoff ersetzbaren Substituenten bedeuten, oder $X_1$ und $X_2$ zusammen einen zweiwertigen, durch zwei Wasserstoffatome ersetzbaren Rest bedeuten, und/oder in den Gruppen R und Ac vorhandene funktionelle Gruppen gegebenenfalls in geschützter Form vorliegen mit der Massgabe, dass mindestens einer der Reste $X_1$ und $X_2$ von Wasserstoff verschieden ist, oder mindestens $X_1$ und $X_2$ zusammen einen zweiwertigen, durch zwei Wasserstoffatome ersetzbaren Rest darstellen, oder mindestens in der Gruppe R und/oder Ac vorhandene funktionelle Gruppen in geschützter Form vorliegen, oder in einem Salz davon, die von Wasserstoff verschiedenen Gruppen $X_1$, $X_2$ oder $X_1$ und $X_2$ zusammen durch Wasserstoffatome ersetzt, und/oder in einer Gruppe R und/oder Ac die an eine oder mehrere funktionelle Gruppen gebundenen Schutzgruppen abspaltet und durch Wasserstoff ersetzt, oder

c) eine Verbindung der Formel

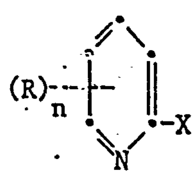

(IIa) ,

worin X eine geeignete Abgangsgruppe darstellt, mit einer Verbindung der Formel

$$H_2N\text{-}NH\text{-}Ac \qquad (V)$$

umsetzt, oder

d) zur Herstellung einer Verbindung der Formel I, worin n für 1 steht, in einer Verbindung der Formel

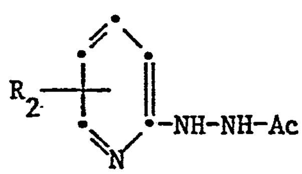 (VI) ,

worin Hydroxy- und/oder Aminogruppen durch Schutzgruppen geschützt sind, und $R_2$ einen zur Bildung eines mono- oder bicyclischen Heteroarylrestes R befähigten Substituenten darstellt, die mono- oder bicyclische Heteroarylgruppe R bildet, gegebenenfalls vorhandene Schutzgruppen gleichzeitig oder nachfolgend abspaltet und durch Wasserstoff ersetzt, und, wenn erwünscht, eine so erhältliche Verbindung der Formel I in eine andere Verbindung der Formel I umwandelt, und/oder, wenn erwünscht, ein erhaltenes Racematgemisch in die Racemate, oder ein erhaltenes Racemat in die optischen Antipoden auftrennt, und/oder, wenn erwünscht, eine erhaltene freie Verbindung der Formel I in ein Salz oder ein erhaltenes Salz in eine freie Verbindung oder ein anderes Salz überführt.

35. Die nach den Verfahren des Anspruchs 34 erhältlichen Verbindungen.

36. Neue Ausgangsstoffe der Formel

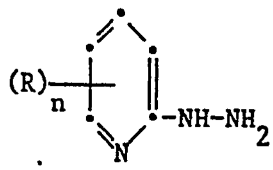

(II) ,

worin R und n die unter der Formel I angegebenen Bedeutungen haben, n insbesondere für 1 steht und R in 3- oder 6-Stellung, insbesondere jedoch in 5-Stellung steht und, Niederalkoxyniederalkyl, z.B. 2-Methoxyäthyl, oder Phenylniederalkyl, z.B. Benzyl, oder falls n 1 ist, Niederalkyl mit 2-7 Kohlenstoffatomen wie Aethyl, n-Propyl oder n-Butyl darstellt, und, mit der weiteren Massgabe dass R als Niederalkyl in 4-Stellung 3-7 Kohlenstoffatome hat, und z.B. n-Propyl oder n-Butyl bedeutet, wenn n 1 ist, oder deren Salze, insbesondere pharmazeutisch verwendbare, nicht-toxische Säureadditionssalze.

FO 7.4/EJA/gb*

BAD ORIGINAL

**0139917**

Nummer der Anmeldung

Europäisches Patentamt

# EUROPÄISCHER RECHERCHENBERICHT

| EINSCHLÄGIGE DOKUMENTE | | | EP 84109311.5 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | US - A - 4 071 632 (J.L. BUTTERFIELD; G.C. WRIGHT)<br>* Gesamt *<br>-- | 1,30, 32,36 | A 61 K 31/44<br>C 07 D 213/77 |
| A | US - A - 4 260 767 (J.P. DUSZA)<br>* Gesamt *<br>-- | 1,7, 32-34 | |
| A | CHEMICAL ABSTRACTS, Band 82, Nr. 25, 23. Juni 1975, Columbus, Ohio, USA<br>M. MOVRIN, D. MAJSINGER "Hydrazine derivatives as potential cytostatics"<br>Seite 512, rechte Spalte, Zusammenfassung-Nr. 170 634v<br>& Eur.J.Med.Chem.-Chim.Ther. 1974, 9(6), 615-617<br>---- | | RECHERCHIERTE SACHGEBIETE (Int. Cl.4)<br>A 61 K 31/00<br>C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 08-10-1984 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82